# EUROPEAN PATENT APPLICATION

(11) **EP 2 163 541 A1**
(43) Date of publication of application: **17.03.2010**
(21) Application number: 08164279.5
(22) Date of filing: 12.09.2008
(51) Int. Cl.: C07D 209/24, C07D 213/81, C07D 213/82, C07D 239/42, C07D 405/12, C07D 295/185, A61K 31/496, A61P 25/00, A61K 51/04

(54) **Piperazine derivatives for binding and imaging amyloid plaques and their use**

(71) Applicant: Bayer Schering Pharma Aktiengesellschaft, 13353 Berlin (DE)
(72) Inventor: Schmitt-Willich, Heribert Dr., 10551, Berlin (DE); Röhn, Ulrike Dr., 10115 Berlin (DE); Friebe, Matthias Dr., 13509 Berlin (DE); Lehmann, Lutz Dr., 42329 Wuppertal (DE); Fitzner, Ansgar Dr., 20253 Hamburg (DE); Krause, Sabine Dr., 13156 Berlin (DE); Brockschnieder, Damian Dr., 10551 Berlin (DE); Dyrks, Thomas Dr., 10625 Berlin (DE); Thiele, Andrea Dr., 13125 Berlin (DE); Bömer, Ulf Dr., 16548 Glienicke/Nordbahn (DE); Mönning Ursula Dr., 15569 Berlin (DE); Heinrich, Tobias Dr., 13507 Berlin (DE)

(57) **Abstract**

The invention relates to compounds of formula I, 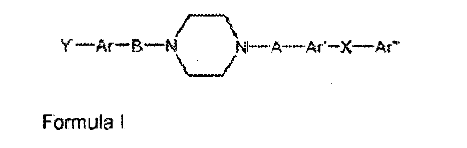 their synthesis and their use, in particular for detecting amyloid deposits in a patient.

## Description

The present invention relates to novel compounds useful for binding and imaging amyloid deposits and their use in detecting or treating Alzheimer's disease and amyloidoses.

### Background of the Invention

Alzheimer's disease (AD) is a progressive neurodegenerative disorder marked by loss of memory, cognition, and behavioral stability. AD is defined pathologically by extracellular senile plaques comprised of fibrillar deposits of the beta-amyloid peptide (Aβ) and neurofibrillary tangles comprised of paired helical filaments of hyperphosphorylated tau. The 39 to 43 amino acids comprising Aβ peptides are derived from the larger amyloid precursor protein (APP). In the amyloidogenic pathway, Aβ peptides are cleaved from APP by the sequential proteolysis of β- and γ-secretases. Aβ peptides are released as soluble proteins and can be detected at low levels in the cerebrospinal fluid (CSF) in normal aging brains. During the progress of AD the Aβ peptides aggregate and form amyloid deposits in the parenchyma and vasculature of the brain, which can be detected post mortem as diffuse and senile plaques and vascular amyloid during histological examination (for a recent review see: Blennow et al. Lancet. 2006 Jul 29;368(9533):387-403).
Alzheimer's disease is becoming a great health and social economical problem all over the world. There are great efforts being made to develop techniques and methods for the early detection and effective treatment of the disease. Currently, diagnosis of AD in an academic setting of memory-disorder clinics is approximately 85-90% accurate (Petrella JR et al. Radiology. 2003 226:315-36). It is based on the exclusion of a variety of diseases causing similar symptoms and the careful neurological and psychiatric examination, as well as neuropsychological testing. However, post mortem histological examination of the brain is still the only definite diagnosis of this disease. Thus the *in vivo* detection of one pathological feature of the disease - the deposition of amyloid aggregates in the brain - is thought to have a big impact on the early detection of AD and differentiation from other dementias. Additionally, most disease modifying therapies that are under development are aiming at lowering the amyloid load in the brain. Thus imaging the amyloid load in the brain may provide an essential tool for patient stratification and treatment monitoring.

In addition, amyloid deposits are also known to play a role in amyloidoses, in which amyloid proteins are abnormally deposited in different organs and/or tissues, causing disease. For a recent review see Chiti et al. Annu Rev Biochem. 2006;75:333-66.

Potential ligands for visualizing amyloid aggregates in the brain must show a high binding affinity to amyloid and must cross the blood brain barrier. PET tracers that have been already investigated in humans regarding their binding patterns in brains of AD patients are [F-18]FDDNP (Shoghi-Jadid et. al, Am J Geriatr Psychiatry 2002; 10:24-35), [C-11]PIB (Klunk e. al, Ann Neurol. 2004 55:306-319), [C-11]SB-13 (Verhoeff et. al, Am J Geriatr Psychiatry 2004; 12:584-595, [F-18]Bay 94-9172 (Rowe et al. Lancet Neurol 2008, 7:129-135), [C-11]BF227 (Kudo et. al, J Nucl. Med 2007; 49:554-561), and [F-18]PIB (Farrar et. al Turku PET Symposium 2007, Abstract 49). For recent reviews see Lockhardt, Drug Discov Today, 2006 11:1093-1099, Henriksen et al., Eur. J. Nucl. Med. Mol. Imaging 2007, Cohen, Mol. Imaging Biol. 2007 9:204-216, Nordberg, Curr. Opin Biol. 2007, 20:398-402, Small et al., Neurology 2008 7:161-172, Nordberg, Eur. J. Nucl. Med. Mol. Imaging 2008, 35, S46-S50.
Besides their specific binding to amyloid deposits in the brain, the currently most promising PET tracers show a disadvantageous non-specific accumulation, especially in white matter brain regions in AD patients as well as in HC. Generally, non-specific background binding interferes with the image quality and could e.g. impair the quantification of amyloid and the diagnosis of very early stages of the disease.
Accordingly, the problem underlying the present invention was to provide compounds suited for detecting amyloid deposits in patients with amyloid-related diseases with high specificity at an early stage of the disease.
The present invention solves this problem by providing novel tracers with high affinity for amyloid β and rapid elimination of non-specific signals from the brain.

### Description of the invention

The present invention is directed to compounds that bind to amyloid deposits and are able to pass through the blood-brain barrier, and are therefore useful in diagnosing Alzheimer's disease and amyloidoses in a patient, preferably at an early stage of the disease.

Accordingly, in one aspect, the invention is directed to compounds according to formula I 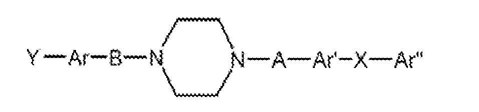 Formula I
and to pharmaceutically acceptable salts or prodrugs thereof,
wherein
- Y is selected from the group consisting of: F, Cl, Br, I, H, detectable labels, such as ¹⁸F, ¹⁹F, ⁷⁶Br, ¹²³I, ¹²⁵I, ¹¹C, ³H, ¹³N, ¹⁵O; leaving groups, such as tosyl, brosyl, nosyl, triflate, sulfonate, substituted sulfonate, mesylate, and nonaflate; and if directly bound to an aromatic C-atom iodonium-aryl I⁺-aryl, trialkylammonium, preferred trimethylammonium, and NO₂;

Ar is selected from the group consisting of:
mono-, bi- or tricyclic aromatic or heteroaromatic ring systems, optionally substituted by one or two alkyl, alkylen, alkynesubstituents and/or alkoxysubstituents,
   wherein the alkyl, alkylen, alkynesubstituents and/or alkoxysubstituents optionally are substituted, wherein the substituent is preferably selected from oxo or hydroxyl,
   and wherein further the alkyl, alkylen, alkynesubstituents and/or alkoxysubstituents may be interrupted by 1 - 5 oxygen atoms, -SO- or -SO₂- groups, preferably the subtituents are polyethylenglycol-moieties,
   and wherein further the alkyl, alkylen, alkynesubstituents and/or alkoxysubstituents may comprise C₃-C₆ cycloalkyl moieties,
   and wherein the mono-, bi- or tricyclic aromatic or heteroaromatic ring systems may be further substituted by electron withdrawing groups directly bound to an aromatic C-atom,
   wherein preferred electron withdrawing groups are -CN or -CF₃;

Ar is preferably selected from the group consisting of: phenyl, 2, 3, or 4 pyridyl, pyrimidyl, pyrazyl, propylpyrimidin-2-yl, ethoxyphenyl, (CH₂CH₂O)₃phenyl, alkylphenyl, alkoxyphenyl, N-alkylindolyl, and alkylpyridyl, and is in an even more preferred embodiment 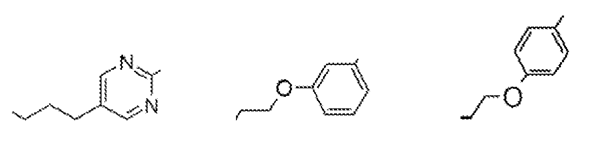 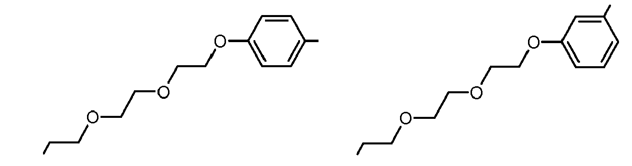 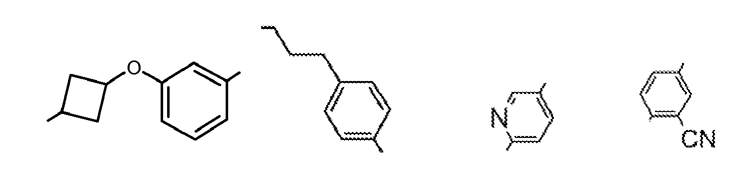 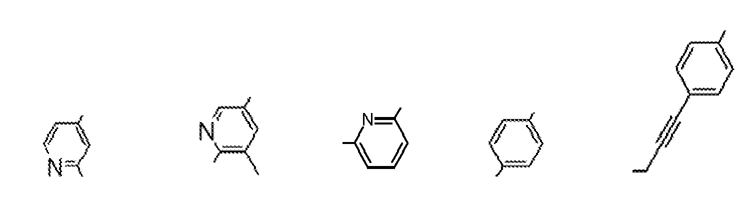 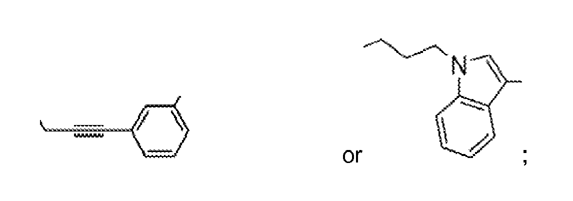
- B is selected from the group consisting of:
   direct bond, a branched or non-branched alkyl or alkylenchain comprised of 1-10 C-atoms wherein the alkylen chain may comprise 1 or 2 unsaturated bonds,
      and wherein the alkyl or alkylenchain is optionally interrupted by N, S, SO, SO₂ or O,
      and wherein the alkyl or alkylenchain is optionally substituted by oxo or -OH;

Preferably, B is selected from the group consisting of
direct bond, CONH-CH₂CO, CO-(CH₂)₂CO, (CH₂)ₙCO, O(CH₂)ₙCO with n=1 to 10, and (CH=CH)CO,
and is even more preferably 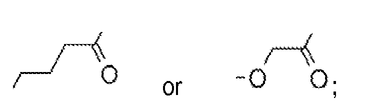
   - A is selected from the group consisting of:
      a direct bond, and CO-NH, CS-NH;
   - Ar' is selected from the group consisting of:
      mono-, or bi-cyclic aromatic or heteroaromatic ring systems, optionally substituted by one or two alkyl, alkylen, alkynesubstituents and/or alkoxysubstituents,
         wherein the alkyl, alkylen, alkynesubstituents and/or alkoxysubstituents optionally are substituted, wherein the substituent is preferably selected from oxo or hydroxyl,
         and wherein further the alkyl, alkylen, alkynesubstituents and/or alkoxysubstituents may be interrupted by 1 - 5 oxygen atoms, preferably the subtituents are polyethylenglycol-moieties,
         and wherein further the alkyl, alkylen, alkynesubstituents and/or alkoxysubstituents may comprise C₃-C₆ cycloalkyl moieties;

Ar' is preferably selected from the group consisting of: phenyl, 2, 3, or 4 pyridyl, pyrimidyl, pyrazyl, propylpyrimidin-2-yl, ethoxyphenyl, (CH₂CH₂O)₃phenyl, alkylphenyl, alkoxyphenyl, N-alkylindolyl, phenyl, benzofuranyl, indolyl and alkylpyridyl,

More preferred, Ar' is selected from the group consisting of
phenyl, benzofuranyl, and indolyl,
and is even more preferably 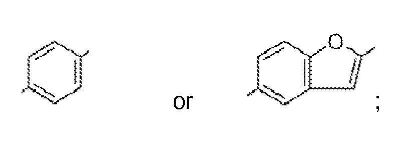 Ar' is mostly preferred phenyl;
X is selected from the group consisting of:
a direct bond or
   a C₁-C₃ alkyl chain, optionally substituted by 1 or 2 substituents that are preferably but not limited to oxo or thio,
   and wherein the alkyl chain may be interrupted by 1 to 2 O, N, S, SO or SO₂ groups;

Preferably, X is selected from the group consisting of
direct bond, OCH₂, NHCO, CH₂O, CONH, NHCS, or CSNH;

Ar" is selected from the group consisting of:
mono-, or bi-cyclic aromatic or heteroaromatic ring systems, optionally substituted by one or two alkyl, alkylen, alkynesubstituents and/or alkoxysubstituents,
   wherein the alkyl, alkylen, alkynesubstituents and/or alkoxysubstituents optionally are substituted, wherein the substituent is preferably selected from oxo or hydroxyl,
   and wherein further the alkyl, alkylen, alkynesubstituents and/or alkoxysubstituents may be interrupted by 1 - 5 oxygen atoms, preferably the subtituents are polyethylenglycol-moieties,
   and wherein further the alkyl, alkylen, alkynesubstituents and/or alkoxysubstituents may comprise C₃-C₆ cycloalkyl moieties.

More preferably. Ar" is selected from te group consisting of
phenyl, 1-phenyl, 1-naphthyl, 2-naphthyl, and all respective heterocyles thereof, and is even more preferably

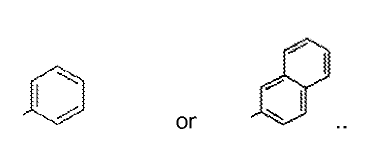

Ar'as well as Ar" can optionally also be substituted by F, Cl, Br, I, H, detectable labels, such as ¹⁸F, ¹⁹F, ⁷⁶Br, ¹²³I, ¹²⁵I, ¹¹C, ³H, ¹³N, ¹⁵O; leaving groups, such as tosyl, brosyl, nosyl, triflate, sulfonate, substituted sulfonate, mesylate, and nonaflate; and if directly bound to an aromatic C-atom iodonium-aryl I⁺-aryl, trialkylammonium, preferred trimethylammonium, and NO₂

For diagnostic purposes, both *in vitro* and *in vivo*, those compound of formula I are preferred that comprise or contain a detectable label, such as a radioactive nuclide or a fluorescent label. For in vitro use, histological sections such as fresh frozen samples or paraffin samples can be analyzed.

Preferred embodiments of the compounds of formula I are given below and are designated compounds 1d/e, 2d/e, 3g/h, 4f/g, 5b/c, 6f, 7d, 8g, 9d/e, 10, 11, 12, 13, 14, 15, 16, 17, 18 ,19, 20, 21 and 22. These preferred embodiments also exemplify the different groups which can be represented by the letters Y, Ar, B, A, Ar', X, and Ar" of formula I.

"Alkyl" refers to a straight or branched chain group consisting solely of carbon and hydrogen, containing no unsaturation and having from one to eight carbon atoms, e.g., methyl, ethyl, n-propyl, 1-methylethyl (iso-propyl), n-butyl, n-pentyl, 1,1-dimethylethyl (t-butyl), n-heptyl, and the like. "Alkoxy" refers to a group of the formula -Oalkyl where alkyl is as defined above.

In the context of the present invention, preferred salts are pharmaceutically acceptable salts of the compounds according to the invention. The invention also comprises salts which for their part are not suitable for pharmaceutical applications, but which can be used, for example, for isolating or purifying the compounds according to the invention.

Pharmaceutically acceptable salts of the compounds according to the invention include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalene disulphonic acid, acetic acid, trifluoroacetic acid, propionic acid, lactic acid, tartaric acid, malic acid, citric acid, fumaric acid, maleic acid and benzoic acid.

Pharmaceutically acceptable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts, derived from ammonia or organic amines having 1 to 16 carbon atoms, such as, by way of example and by way of preference, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

Moreover, the present invention also comprises prodrugs of the compounds according to the invention. The term "prodrugs" includes compounds which for their part may be biologically active or inactive but which, during the time they spend in the body, are converted into compounds according to the invention (for example metabolically or hydrolytically).

In particular, the present invention also comprises hydrolyzable ester derivatives of the carboxylic acids of the formula (I). These are to be understood as being esters which can be hydrolyzed in physiological media and in particular *in vivo* by enzymatical or chemical means to give the free carboxylic acids. Such esters are preferably straight-chain or branched (C₁-C₆)-alkyl esters in which the alkyl group may be substituted by hydroxyl, (C₁-C₄)-alkoxy, amino, mono-(C₁-C₄)-alkylamino and/or di-(C₁-C₄)-alkylamino. Particular preference is given to the methyl or ethyl esters of the compounds of the formula (I).
In the context of the present invention, unless specified otherwise, the substituents have the following meanings:
In the context of the invention, alkyl represents a straight-chain or branched alkyl radical having the number of carbon atoms stated in each case. The following radicals may be mentioned by way of example and by way of preference: methyl, ethyl, n-propyl, isopropyl, n-butyl, isobutyl, 1-methylpropyl, tert-butyl, n-pentyl, isopentyl, 1-ethylpropyl, 1-methylbutyl, 2-methylbutyl, 3-methylbutyl, n-hexyl, 1-methylpentyl, 2-methylpentyl, 3-methylpentyl, 4-methylpentyl, 3,3-dimethylbutyl, 1-ethylbutyl, 2-ethylbutyl, 1,4-dimethylpentyl, 4,4-dimethylpentyl and 1,4,4-trimethylpentyl.
In the context of the invention, cycloalkyl represents a monocyclic saturated alkyl radical having 3 to 7 carbon atoms. The following radicals may be mentioned by way of example and by way of preference: cyclopropyl, cyclobutyl, cyclopentyl, cyclohexyl and cycloheptyl.
In the context of the invention, alkoxy represents a straight-chain or branched alkoxy radical having 1 to 4 carbon atoms. The following radicals may be mentioned by way of example and by way of preference: methoxy, ethoxy, n-propoxy, isopropoxy, 1-methylpropoxy, n-butoxy, isobutoxy and tert-butoxy.
In the context of the invention, halogen includes fluorine, chlorine, bromine and iodine. Preference is given to fluorine.

If radicals in the compounds according to the invention are substituted, the radicals can, unless specified otherwise, be mono- or polysubstituted. In the context of the present invention, the meanings of all radicals which occur more than once are independent of one another.

Substitution with one, two or three identical or different substituents is preferred. Very particular preference is given to substitution with one substituent.

The compounds of the invention, or their pharmaceutically acceptable salts, may have asymmetric carbon atoms in their structure. The compounds of the invention and their pharmaceutically acceptable salts may therefore exist as single enantiomers, diastereoisomers, racemates, and mixtures of enantiomers and diastereomers. All such single enantiomers, diastereoisomers, racemates, and mixtures thereof are within the scope of this invention.

The compounds as described above and herein are, in a preferred embodiment of the invention, bound to an Aβ peptide.

Another aspect of the invention is the use of a compound of formula I as described above and herein for diagnosing and/or treating Alzheimer's disease and/or amyloidoses in a patient, in particular in a mammal, such as a human.

The treatment of a patient with Alzheimer's disease and/or amyloidoses can preferably be performed with a compound of the invention according to formula I that does not bear a radioactive label, but in which Y is e.g. hydrogen.

Preferably, the use of a compound of the invention in the diagnosis is performed using positron emission tomography (PET), single photon emission computed tomography (SPECT), magnetic resonance (MR)-spectoscropy or tomography.

Another aspect of the invention is directed to a method of imaging amyloid deposits. Such a method comprises a) administering to a mammal a compound as described above and herein containing a detectable label, and b) detecting the signal stemming from the compound that is specifically bound to the amyloid deposits. The specific binding is a result of the high binding affinity of the compounds of the present invention to the amyloid deposits.

In a further aspect, the invention is directed to a method of diagnosing a patient with Alzheimer's disease or amyloidoses. This method comprises a) administering to a human in need of such diagnosis a compound of the invention with a detectable label for detecting the compound in the human as described above and herein, and b) measuring the signal from the detectable label arising from the administration of the compound to the human, preferably by using a gamma camera, by positron emission tomography (PET), or by single photon emission computed tomography (SPECT).

A further embodiment of the invention includes a diagnostic method for other neurological disorders as Alzheimer's disease comprising the exclusion of Alzheimer's disease in a patient, that method comprising administering a compound of the invention to a patient and applying an imaging method of the invention.

A further aspect of the invention refers to a diagnostic composition for imaging amyloid deposits, comprising a radiolabeled compound according to formula I.

The diagnostic methods of the invention can also be used as post-mortem diagnostic methods.

Furthermore, the diagnostic methods of the invention can also be used for monitoring the therapy of Alzheimer's disease, a neurodegenerative disorder or an amyloidoses.

Furthermore, the diagnostic methods of the invention can also be used in diagnosing neurological disorders other than Alzheimer's disease by excluding Alzheimer's disease.

In a further aspect of the invention, the invention comprises a method of treating or preventing amyloidoses or Alzheimer's disease comprises administering to a human in need of such a treatment a compound of formula I as described herein.

A further aspect of the invention refers to a pharmaceutical composition which comprises a compound of the invention as described herein, optionally together with a suitable carrier and/or additive.

Furthermore, the compounds of the invention can also be used as tools in screening, for example high throughput screening methods and in vitro assays.

The invention also refers to a method for synthesizing a compound of the invention according to formula I as described herein. The general synthetic methods of the compounds of the invention are as follows.

### F-18 radiolabeling

A further aspect of the invention refers to a method of radiofluorination of a compound of formula I for the manufacture of radiolabeled compound of formula I comprising the step of reacting a compound of formula I with a fluorination agent. Useful Radiofluorination methods are well known to the person skilled in the art.

In a preferred embodiment, the fluorination agent is 4,7,13,16,21,24-Hexaoxa-1,10 diazabicyclo[8.8.8]-hexacosane K¹⁸F (crownether salt Kryptofix K¹⁸F), K¹⁸F, H¹⁸F, KH¹⁸F₂ or tetraalkylammonium salt of ¹⁸F. More preferably, fluorination agent is K¹⁸F, H¹⁸F, or KH¹⁸F₂.

The solvents used can be Dimethylformamide, DMF, Dimethylsulfoxyde, DMSO, Acetonitrile, MeCN, Dimethylacetamide, DMA, DMAA etc., preferably DMSO, MeCN or DMF. The solvents can also be a mixture of solvents as indicated above.

[F-18] radiolabeling procedures are well known to the person skilled in the art. For example, radiolabeling can be preformed as described in the following.

[F-18]Fluoride can be produced by proton bombardment in a cyclotron using a silver target (1 mL) filled with [O-18] water for the ¹⁸O (p,n)¹⁸F reaction. The aqueous [F-18]fluoride can be passed through a cartridge (e.g. QMA-resin cartridge Waters, Sep Pak Light QMA Part.No.: WAT023525 ). The trapped [F-18]fluoride can then be eluted from the cartridge by adding e.g. a Kryptofix K2.2.2/ K₂CO₃ solution (Kryptofix is 4,7,13,16,21,24-Hexaoxa-1,10-diazabicyclo[8.8.8]-hexacosane). The nucleophilic substitution of the precursor works preferably in the presence of a base such as NBu₄OH, (NBu₄)₂CO₃, K₂CO₃ etc. and at elevated temperatures. The addition of crown ethers such as Kryptofix (K2.2.2) can influence the reaction positively, especially in the presence of K₂CO₃ as the base.

The potassium fluoride Kryptofix complex is preferably dried by repeated azeotropic distillation with sequential addition of acetonitrile. Solvents such as acetonitrile, DMF, DMSO etc. can be used as a reaction solvent. The labeling product can be purified by solid phase extraction using cartridges. Preferred cartridges are Sep-Pak Plus C18 cartridge (Waters, WAT020515). The cartridge can be rinsed with water and the compound can be eluted with acetonitrile. The eluted compound can be diluted with water and can then be subjected to preparative HPLC purification. Preferred HPLC columns are reversed phase columns such as Gemini 5 µ C 18 110 Å, 250 * 10 mm (Phenomenex, 00G-4435-N0). Mixtures of buffer solution, acids, water etc. with organic solvents such as acetonitrile, methanol, ethanol etc. can be used as mobile.

The solution can then be diluted with e.g. water to be passed through a cartridge for concentration and solvent change.

### General synthesis of F-18 compounds: alkyl-F and (hetero)aryl-F

Precursors for alkyl-F-18 compounds of general formula I (formula I with Y = ¹⁸F) are e.g. tosylates, brosylates, nosylates, mesylates, triflates, nonaflates etc. (formula I with Y = leaving group) which can be synthesized from the respective hydroxy compounds according to methods known in the art (J. March, Advanced Organic Chemistry, 4th ed. 1992, John Wiley & Sons, pp 352ff). An additional method is described in Examples 3f, 4e and 5a and comprises the synthesis by suitable bis(tosylates) and the like, e.g. TsO-(CH₂)ₙ-OTs.

Other precursors for alkyl-F-18 compounds of general formula I (formula I with Y = ¹⁸F) are e.g. iodides and bromides and the like whose conversion to the respective fluorides is also known in the art (J. March, see above).

Precursors for aryl-F-18 compounds of general formula I are e.g. aryl or heteroaryl bromides, nitro compounds, trialkyl ammonium, aryliodonium which can be converted to the respective F-18 compounds of this invention by methods known in the art (L. Cai, S. Lu, V. Pike, Eur. J. Org. Chem 2008, 2853-2873). Starting materials for these precursors are commercially available or can be synthesized by methods known in the art (R.C. Larock, Comprehensive Organic Transformations, VCH Publishers 1989).

The synthesis of hydroxy compounds as starting materials for tosylates, brosylates, nosylates, mesylates, triflates, nonaflates etc. comprises
- the deprotection of OH-protecting groups. As one of the very versatile protecting groups might be mentioned the acetyl protecting group. Many others are known in the art, see e.g. T.W. Greene and P.G.M. Wuts, Protective Groups in Organic Synthesis, 3rd ed, 1999, John Wiley & Sons, pp 17ff), see also e.g. Examples 3d and 4c
   or
- the introduction of the hydroxyalkyl group by Pd catalyzed substitution of 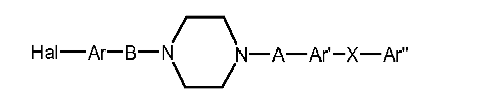 with Hal = Br,I as described in Examples 1b and 2b
   or
- as in Example 6d, where the hydroxy compound HO-Ar-B' with the meaning of B' as - CO-(CH₂)ₙ-COOH can be synthesized by Friedel Crafts Acylation.

Compounds of the general formula I 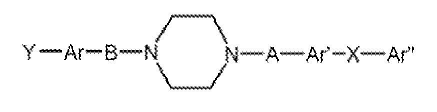 with Y = Br, I, HO, Protecting Group-O- can be synthesized by
- an amide coupling to establish -CO-NH- groups in any position and sequence order
   or
- optionally by reaction of a piperazine derivative with a suitable amino component by use of phosgene or an equivalent to phosgene to build a urea bond (-NH-CO-NH-) as described in Examples 1a and 2a.

Aryl-piperazines are commercially available or can be synthesized according to the literature, e.g. according to Klapars et a/., Journal of the American Chemical Society 2002, 124, 7421-28 *and literature cited herein.*

A further aspect of the invention refers to a kit comprising a non-radiolabeled compound of the invention, the compound optionally being in a dry condition or having an inert, pharmaceutically acceptable carrier and/or solvent and/or auxiliary substances added. In a preferred embodiment the kit comprises one or more sealed containers which comprise the compounds of the kit. In a preferred embodiment the kit comprises a compound of formula I as described herein, in which Y is a leaving group, such as tosyl, brosyl, nosyl, triflate, sulfonate, substituted sulfonate, mesylate, nonaflate, iodonium-aryl I⁺-aryl, trialkylammonium, trimethylammonium, or NO₂.

Yet another aspect of the invention refers to a method of inhibiting the formation of amyloid or modulating the pathogenicity of amyloid in a mammal. This method comprises administering a compound of formula I as described herein in an amount that is effective to inhibit the formation of amyloid or to modulate the pathogenicity of amyloid.

### Preferred Compounds of the Invention

Of the various aspects of the invention, certain compounds of formula I are preferred. Such preferred compounds are given below, wherein those compounds are named with a figure together with a letter which refer to the examples given below describing the synthesis of these compounds.

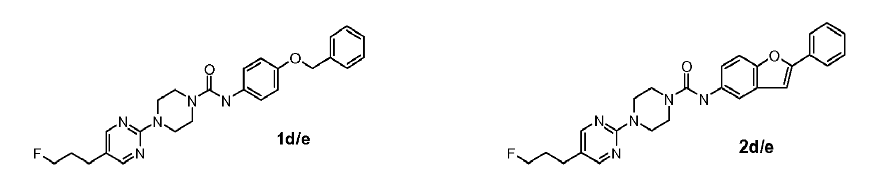

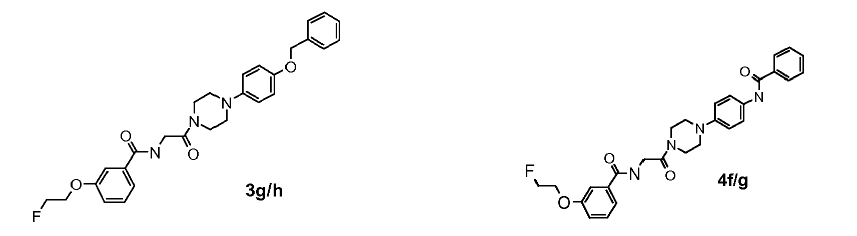

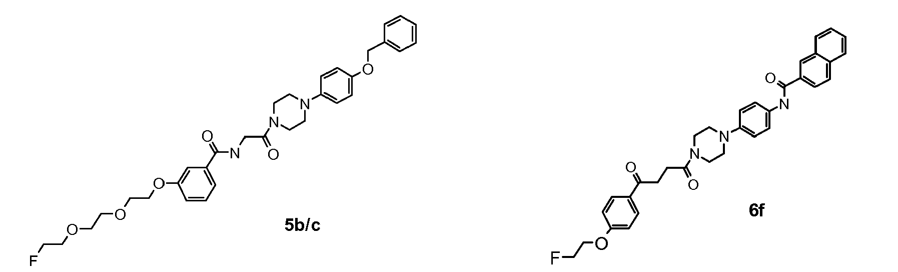

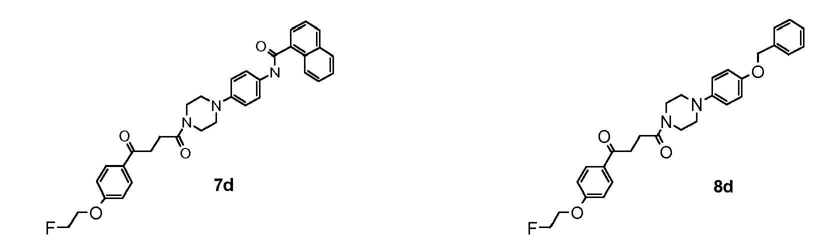

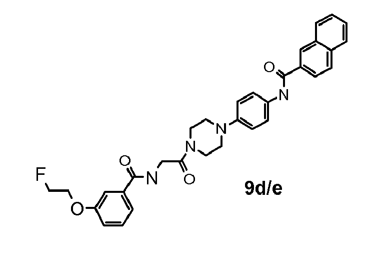

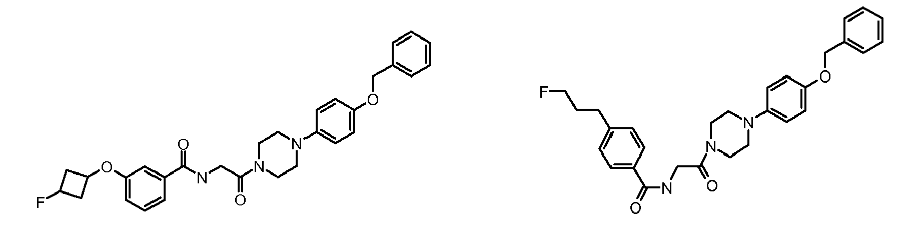

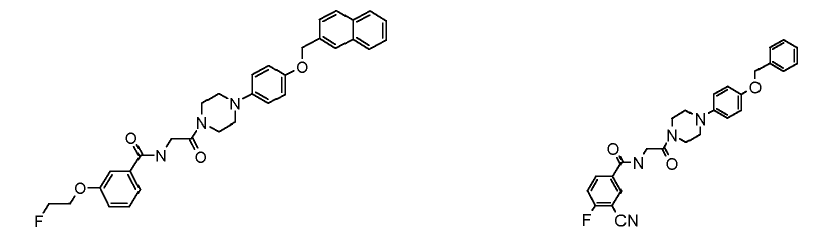

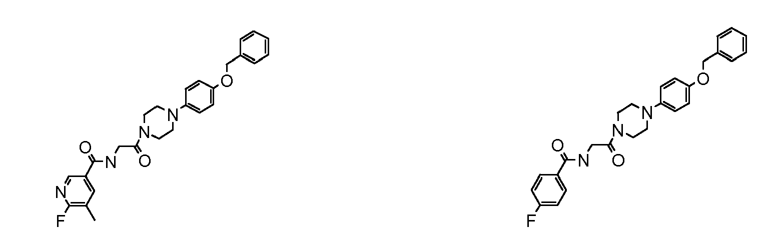

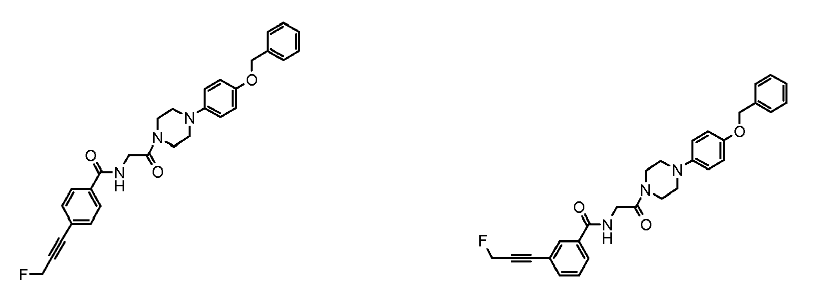

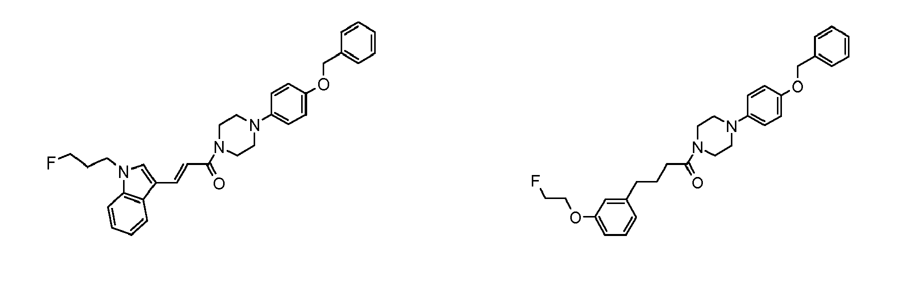

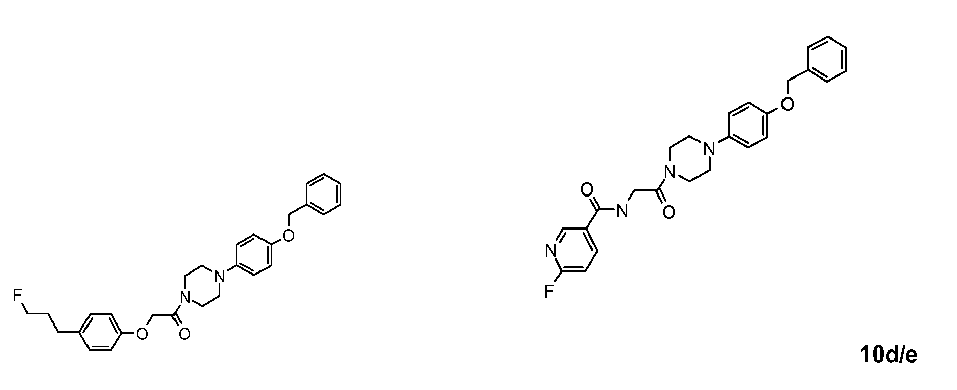

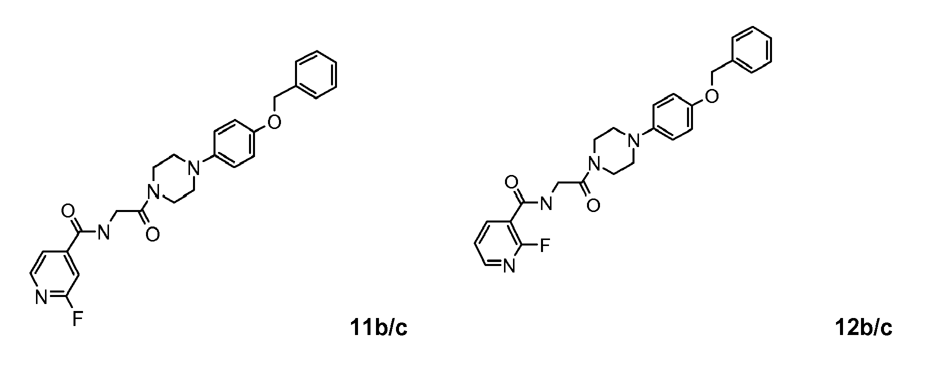

Even more preferred embodiments of the invention are the respective F-18 compounds.

The compounds of the invention represent novel tracers with high affinity for amyloid β and rapid elimination of non-specific signals from the brain.

### Brief description of the Figures

Fig. 1 shows the autoradiographical analysis of binding of 3g to cryosections from cortex of Alzheimer's disease patients (AD) and controls without Aβ plaques (HC/FTD) (healthy control/ frontotemporal dementia). Specific binding in plaque-rich regions of AD samples is indicated by arrows.
Fig. 2 shows the autoradiographical analysis of binding of example 10e to cryosections from cortex of Alzheimer's disease patients (AD) and controls without Aβ plaques (HC/FTD) (healthy control/ frontotemporal dementia). Specific binding in plaque-rich regions of AD samples is indicated by arrows.
Fig. 3 shows the Autoradiographical analysis of binding of example 11c to cryosections from cortex of Alzheimer's disease patients (AD) and controls without Aβ plaques (HC/FTD) (healthy control/ frontotemporal dementia). Specific binding in plaque-rich regions of AD samples is indicated by arrows.
Fig. 4 shows the autoradiographical analysis of binding of example 12c to cryosections from cortex of Alzheimer's disease patients (AD) and controls without Aβ plaques (HC/FTD) (healthy control/ frontotemporal dementia). Specific binding in plaque-rich regions of AD samples is indicated by arrows.
Fig. 5 shows IC50 values in [nM] of selected compounds measured in a competition assay using brain homogenate from AD patients.

### Examples

The methods for synthesizing and labeling these compositions are more fully illustrated in the following Examples. These Examples illustrate certain aspects of the above-described method and advantageous results and are shown by way of illustration and not by way of limitation.

### Example 1

### a) 4-(5-Bromo-pyrimidin-2-yl)-piperazine-1-carboxylic acid (4-benzyloxy-phenyl)-amide

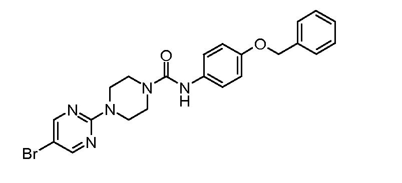

A solution of 707 mg (3 mmol) of 4-benzyloxy-phenylamine hydrochloride (Aldrich) and 1.13 mL (6.6 mmol) of N,N-ethyl diisopropylamine in 10 mL of dichloromethane are added dropwise to a solution of 297 mg (1 mmol) of triphosgene in 10 mL of dichloromethane at 0°C. The reaction mixture is stirred for 15 minutes at 0°C, then a mixture of 0.73 g (3 mmol) of 5-bromo-2-piperazin-1-yl-pyrimidine and 1.13 mL (6.6 mmol) of N,N-ethyl diisopropylamine in 10 mL of dichloromethane are added. After 30 min at 0°C and overnight at room temperature, the solvent is evaporated and the residue is chromatographed on silica gel using a dichloromethane/ethyl acetate gradient. Yield: 0.93 g (66 %)

| Elemental analysis: | | | | |
|---|---|---|---|---|
| calcd.: | C 56.42 | H 4.73 | Br 17.06 | N 14.95 |
| found: | C 56.11 | H 4.83 | Br 16.94 | N 14.77 |

### b) 4-[5-(3-Hydroxy-propyl)-pyrimidin-2-yl]-piperazine-1-carboxylic acid (4-benzyloxyphenyl)-amide

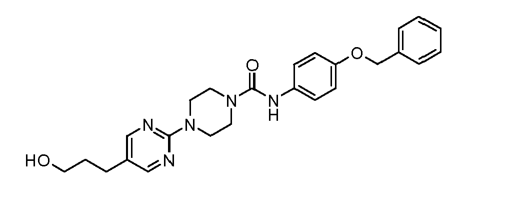

To a solution of 0.21 mL (3 mmol) of allyl alcohol in 9 mL of dry tetrahydrofurane (THF) are added at 0°C 18 mL (9 mmol) of a 0.5 M solution of 9-borabicyclo-(3.3.1)-nonane in THF. This mixture is stirred at 0°C for additional 15 min and 5 h at room temperature (= Solution A).

0.70 g (1.5 mmol) of the bromo derivative compound 1a are suspended in 10 mL dry DMF and 0.35 g (0.3 mmol) of tetrakis(triphenyl phosphine) palladium(0) and 4 mL (12 mmol) 3 M aqueous potassium carbonate solution are added (= Suspension B).
Solution A is added to suspension B and stirred overnight at 65°C. After evaporation of the solvents the residue is chromatographed on silica gel using a dichloromethane/methanol gradient.
Yield: 110 mg (16 %)

| Elemental analysis: | | | |
|---|---|---|---|
| calcd.: | C 67.10 | H 6.53 | N 15.65 |
| found: | C 66.92 | H 6.41 | N 15.88 |

### c) Toluene-4-sulfonic acid 3-{2-[4-(4-benzyloxy-phenylcarbamoyl)-piperazin-1-yl]-pyrimidin-5-yl}-propyl ester

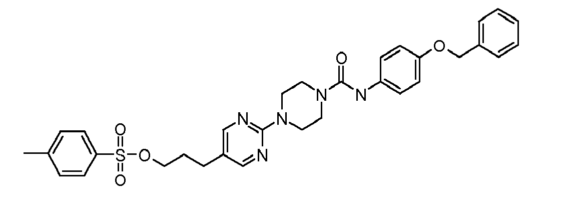

To a suspension of 0,45 g (1 mmol) of the hydroxy derivative 1b in 3 mL of dry pyridine is added dropwise at 0°C a solution of 0.25 g (1.3 mmol) of p-toluenesulfonyl chloride in 2 mL of dry pyridine. After stirring for 30 min the mixture is evaporated to dryness and the residue is chromatographed on silica gel using a dichloromethane/hexane gradient. Yield: 379 mg (63 %)

| Elemental analysis: | | | | |
|---|---|---|---|---|
| calcd.: | C 63.88 | H 5.86 | N 11.64 | S 5.33 |
| found: | C 63.67 | H 5.97 | N 11.48 | S 5.02 |

### d) 4-[5-(3-[F-18]Fluoro-propyl)-pyrimidin-2-yl]-piperazine-1-carboxylic acid (4-benzyloxyphenyl)-amide

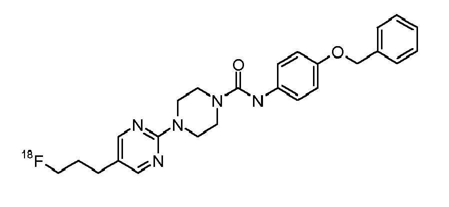

[F-18]Fluoride was produced by proton bombardment in a cyclotron using a low volume, low pressure silver target (1 mL) filled with [O-18] water for the ¹⁸O (p,n)¹⁸F reaction. The aqeous [F-18]fluoride (app. 1 mL) was passed through a QMA-resin cartridge (Waters, Sep Pak Light QMA Part.No.: WAT023525 ). The trapped [F-18]fluoride was eluted from the cartridge by adding a Kryptofix K2.2.2/ K₂CO₃ solution (5 mg K2.2.2/ 1.5 mL acetonitrile + 1 mg K₂CO₃/ 0.5 mL water). The mixture was dried by repeated (2 x) azeotropic distillation with sequential addition of 1 mL of acetonitrile. The tosylate precursor 1c was dissolved in dry dimethylformamide (100 µL) and acetonitrile (400 µL) and added to the dry potassium/ Kryptofix/ fluoride complex. The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0 was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.8 min. Radiochemical yield (RCY) decay corrected (d.c.) at end of bombardment: 44 %. Radiochemical purity (RCP): 98 %.

### e) 4-[5-(3-Fluoro-propyl)-pyrimidin-2-yl]-piperazine-1-carboxylic acid (4-benzyloxy-phenyl)-amide (HPLC standard)

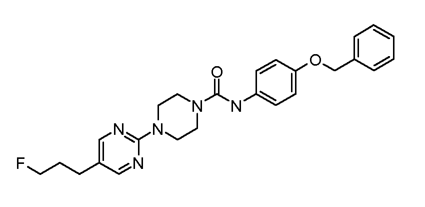

To 67 mg (0.15 mmol) of hydroxy derivative compound 1b in 10 mL dichloromethane were added 48 mg (0.3 mmol) of diethylamino sulfur trifluoride (DAST) at 0°C. After 30 min at 0°C, water was added, the organic phase was dried over sodium sulfate and after evaporation of the organic solvent, the residue was chromatographed on silica gel using a dichloromethane/ethyl acetate gradient. Yield: 40 mg (60 %)

| Elemental analysis: | | | | |
|---|---|---|---|---|
| calcd.: | C 66.80 | H 6.28 | F 4.23 | N 15.58 |
| found: | C 66.54 | H 6.49 | F 4.06 | N 15.77 |

### Example 2

### a) 4-(5-Bromo-pyrimidin-2-yl)-piperazine-1-carboxylic acid (2-phenyl-benzofuran-5-yl)-amide

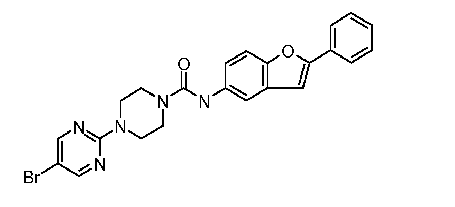

A solution of 628 mg (3 mmol) of 2-phenyl-benzofuran-5-ylamine hydrochloride (ChemBridge Corp., San Diego, USA) and 1.13 mL (6.6 mmol) of N,N-ethyl diisopropylamine in 20 mL of dichloromethane are added dropwise to a solution of 297 mg (1 mmol) of triphosgene in 10 mL of dichloromethane at 0°C. The reaction mixture is stirred for 30 minutes at 0°C, then a mixture of 0.73 g (3 mmol) of 5-bromo-2-piperazin-1-yl-pyrimidine and 0.56 mL, 3.3 mmol) of N,N-ethyl diisopropylamine in 20 mL of dichloromethane are added. After 1 h at 0°C and overnight at room temperature, the solvent is evaporated and the residue is chromatographed on silica gel using a dichloromethane/ethyl acetate gradient. Yield: 0.81 g (56 %)

| Elemental analysis: | | | | |
|---|---|---|---|---|
| ber.: | C 57.75 | H 4.21 | Br 16.70 | N 14.64 |
| gef.: | C 57.43 | H 4.37 | Br 16.11 | N 14.45 |

### b) 4-[5-(3-Hydroxy-propyl)-pyrimidin-2-yl]-piperazine-1-carboxylic acid (2-phenyl-benzofuran-5-yl)-amide

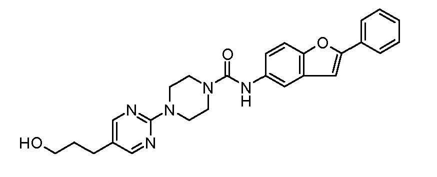

To a solution of 0.21 mL (3 mmol) of allyl alcohol in 9 mL of dry tetrahydrofurane (THF) are added at 0°C 18 mL (9 mmol) of a 0.5 M solution of 9-borabicyclo-(3.3.1)-nonane in THF. This mixture is stirred at 0°C for additional 15 min and 5 h at room temperature (= Solution A).

0.72 g (1.5 mmol) of compound 2a are suspended in 10 mL dry DMF and 0.35 g (0.3 mmol) of tetrakis(triphenyl phosphine) palladium(0) and 4 mL (12 mmol) 3 M aqueous potassium carbonate solution are added (= Suspension B).
Solution A is added to suspension B and stirred overnight at 65°C. After evaporation of the solvents the residue is chromatographed on silica gel using a dichloromethane/methanol gradient. Yield: 85 mg (12 %)

| Elemental analysis: | | | |
|---|---|---|---|
| calcd.: | C 68.25 | H 5.95 | N 15.31 |
| found: | C 68.05 | H 6.12 | N 15.20 |

### c) Toluene-4-sulfonic acid 3-{2-[4-(2-phenyl-benzofuran-5-ylcarbamoyl)-piperazin-1-yl]-pyrimidin-5-yl}-propyl ester

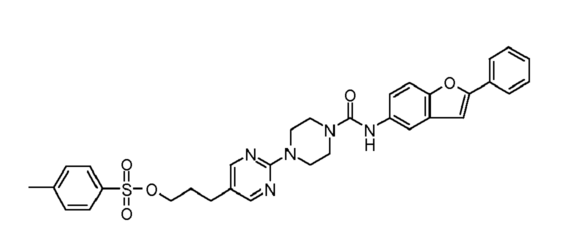

To a suspension of 0,46 g (1 mmol) of the hydroxy derivative 2b in 3 mL of dry pyridine is added dropwise at 0°C a solution of 0.25 g (1.3 mmol) of p-toluenesulfonyl chloride in 2 mL of dry pyridine. After stirring for 30 min the mixture is evaporated to dryness and the residue is chromatographed on silica gel using a dichloromethane/hexane gradient. Yield: 434 mg (71 %)

| Elemental analysis: | | | | |
|---|---|---|---|---|
| calcd.: | C 64.80 | H 5.44 | N 11.45 | S 5.24 |
| found: | C 64.66 | H 5.23 | N 11.74 | S 4.97 |

### d) 4-[5-(3-[F-18]Fluoro-propyl)-pyrimidin-2-yl]-piperazine-1-carboxylic acid (2-phenyl-benzofuran-5-yl)-amide

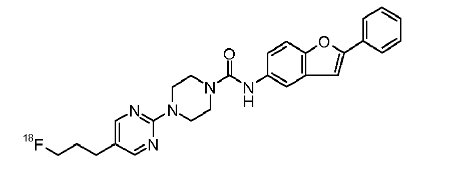

The tosylate precursor 2c was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.1 min. RCY (d.c.): 38 %. RCP: 97 %.

### e) 4-[5-(3-Fluoro-propyl)-pyrimidin-2-yl]-piperazine-1-carboxylic acid (2-phenyl-benzofuran-5-yl)-amide (HPLC standard)

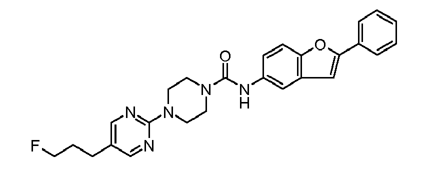

To 69 mg (0.15 mmol) of hydroxy derivative compound 2b in 10 mL dichloromethane were added 48 mg (0.3 mmol) of diethylamino sulfur trifluoride (DAST) at 0°C. After 30 min at 0°C, water was added, the organic phase was dried over sodium sulfate and after evaporation of the organic solvent, the residue was chromatographed on silica gel using a dichloromethane/ethyl acetate gradient.
Yield: 15 mg (22 %)

| Elemental analysis: | | | | |
|---|---|---|---|---|
| calcd.: | C 67.96 | H 5.70 | F 4.13 | N 15.24 |
| found: | C 67.67 | H 5.96 | F 3.96 | N 15.22 |

### Example 3

### a) (3-Acetoxy-benzoylamino)-acetic acid benzyl ester

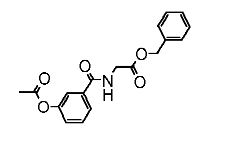

30.4 g (90 mmol) Glycine benzylester p-toluene sulfonate salt are solved in the two phase system dichloromethane and aqueous saturated sodium hydrogencarbonate solution. The organic phase is dried over magnesium sulfate and then evaporated.

13.09 g (79.25 mmol) of free amine is obtained which is used in the subsequent coupling reaction without further purification.
To a solution of 14.28 g (79.25 mmol) 3-Acetoxy-benzoic acid in 150 mL THF and 11 mL triethyl amine (79.25 mmol) at -15°C, 11.39 mL (87.2 mmol) isobutyl chloroformate are added dropwise and the solution is maintained at this temperature for another 15 min. Then, 13.09 g of glycine benzyl ester and 11 mL triethyl amine (79.25 mmol) in 50 mL THF and 50 mL dichloromethane are added slowly to this cold solution, the temperature is kept below 10°C for another 15 min and is then allowed to reach room temperature. After stirring overnight the solvent is evaporated and the residue is chromatographed on silica gel using an ethyl acetate/ethanol gradient.
Yield: 24.6 g (95 %).

| Elemental analysis: | | | |
|---|---|---|---|
| calcd.: | C 66.05 | H 5.23 | N 4.28 |
| found: | C 65.84 | H 5.43 | N 4.16 |

### b) (3-Acetoxy-benzoylamino)-acetic acid

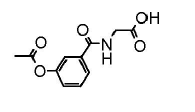

To a solution of 19.64 g (60 mmol) of benzyl ester 3a in 300 mL methanol are added 3 g Pd on charcoal (10%) and the suspension is stirred under hydrogen overnight at room temperature. The catalyst is filtered off and the solvent evaporated.
Yield: 14.2 g (quantitative).

| Elemental analysis: | | | |
|---|---|---|---|
| calcd.: | C 55.70 | H 4.67 | N 5.90 |
| found: | C 55.66 | H 4.49 | N 5.77 |

### c) 1-(4-Benzyloxy-phenyl)-piperazine

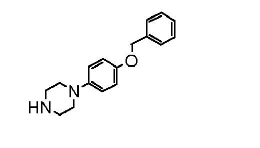

All glassware is dried at 100°C. To a solution of 4.32 g (50.16 mmol) of piperazine in 60 mL toluene is added 459 mg (0.5 mmol) of tris(dibenzylidene acetone) dipalladium(0) and 423 mg (0.68 mmol) of BINAP (2,2'-bis(diphenylphosphino)-1,1'-binaphthyl). Then, a solution of 12 g (45.6 mmol) of 4-Benzyloxy-bromobenzene in 40 mL THF is added followed by a suspension of 6.56 g (68.27 mmol) of sodium t-butylate in THF.
The reaction mixture is refluxed for 3 h and stirred at room temperature overnight. After evaporation of the solvents the residue is chromatographed on silica gel using a dichloromethane/methanol gradient.
Yield: 12.2 g (45.7 %).

| Elemental analysis: | | | |
|---|---|---|---|
| calcd.: | C 76.09 | H 7.51 | N 10.44 |
| found: | C 75.91 | H 7.76 | N 10.20 |

### d) Acetic acid 3-{2-[4-(4-benzyloxy-phenyl)-piperazin-1-yl]-2-oxo-ethylcarbamoyl}-phenyl ester

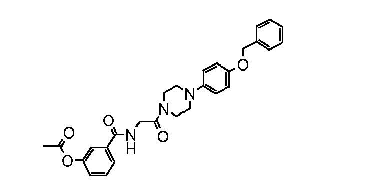

To a solution of 654 mg (2.76 mmol) (3-Acetoxy-benzoylamino)-acetic acid 3b in 70 mL THF and 0.40 mL triethyl amine (2.87 mmol) at -15°C, 0.396 mL (3.03 mmol) isobutyl chloroformate are added dropwise and the solution is maintained at this temperature for another 15 min. Then, 740 mg of 1-(4-benzyloxy-phenyl)-piperazine 3c and 1.7 mL triethyl amine (12.25 mmol) in 30 mL THF and 30 mL dichloromethane are added slowly to this cold solution, the temperature is kept below 10°C for another 15 min and is then allowed to reach room temperature. After stirring overnight the solvent is evaporated and the residue is chromatographed on silica gel using a hexane/ethyl acetate gradient.
Yield: 390 mg (30 %).

| Elemental analysis: | | | |
|---|---|---|---|
| calcd.: | C 68.98 | H 6.00 | N 8.62 |
| found: | C 69.09 | H 5.81 | N 8.42 |

### e) N-{2-[4-(4-Benzyloxy-phenyl)-piperazin-1-yl]-2-oxo-ethyl}-3-hydroxy-benzamide

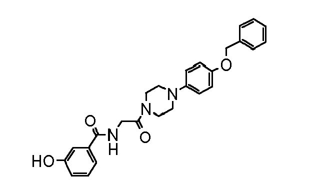

230 mg (0.47 mmol) of the acetate 3d are solved in 30 mL of ethanol and cooled to 0°C. After addition of 1.5 mL 3N NaOH the solution is stirred for 1 h, glacial acetic acid is added until the pH is below pH 7 and the solvents are evaporated. The raw product is crystallized from ethanol.
Yield: 200 mg (95 %).

| Elemental analysis: | | | |
|---|---|---|---|
| calcd.: | C 70.10 | H 6.11 | N 9.43 |
| found: | C 69.88 | H 6.27 | N 9.20 |

### f) Toluene-4-sulfonic acid 2-(3-{2-[4-(4-benzyloxy-phenyl)-piperazin-1-yl]-2-oxo-ethylcarbamoyl}-phenoxy)-ethyl ester

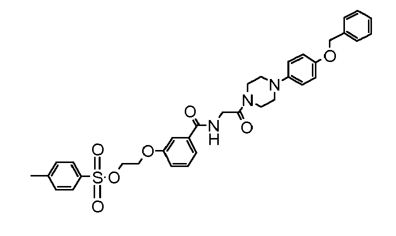

To a solution of 80 mg (0.18 mmol) of N-{2-[4-(4-Benzyloxy-phenyl)-piperazin-1-yl]-2-oxoethyl}-3-hydroxy-benzamide (3e) in 10 mL DMF are added 62 mg (0.45 mmol) of potassium carbonate and 100 mg (0.27 mmol) of 1,2 bis(tosyloxy)ethane (Aldrich) . The mixture is heated to 60°C for 3 h. After stirring overnight at room temperature the solvent is evaporated and the residue is chromatographed on silica gel using an ethyl acetate/hexane gradient.
Yield: 56 mg (48 %).

The compound has a purity >95% according to HPLC and is suitable as a precursor of the F-18 labelling.

### g) N-{2-[4-(4-Benzyloxy-phenyl)-piperazin-1-yl]-2-oxo-ethyl}-3-(2-[F-18]fluoro-ethoxy)-benzamide

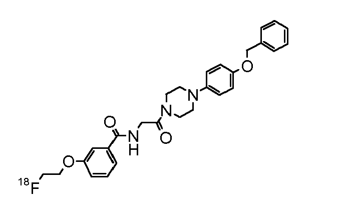

The tosylate precursor 3f was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.3 min. RCY (d.c.): 38 %. RCP: 99 %.

### h) N-{2-[4-(4-Benzyloxy-phenyl)-piperazin-1-yl]-2-oxo-ethyl}-3-(2-fluoro-ethoxy)-benzamide (HPLC standard)

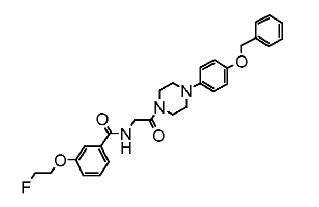

To a solution of 50 mg (0.11 mmol) of N-{2-[4-(4-Benzyloxy-phenyl)-piperazin-1-yl]-2-oxoethyl}-3-hydroxy-benzamide (3e) in 5 mL DMF are added 34 mg (0.25 mmol) of potassium carbonate and 10 uL (0.13 mmol) of 1-fluoro-2-bromoethane (ABCR, Germany). The mixture is heated to 60°C for 3 h. The solvent is evaporated and the residue is chromatographed on silica gel using an ethyl acetate/hexane gradient.
Yield: 30 mg (54 %).

| Elemental analysis: | | | | |
|---|---|---|---|---|
| calcd.: | C 68.42 | H 6.15 | F 3.86 | N 8.55 |
| found: | C 68.17 | H 6.28 | F 3.64 | N 8.71 |

### Example 4

### a) Acetic acid 3-{2-[4-(4-nitrophenyl)-piperazin-1-yl]-2-oxo-ethylcarbamoyl}-phenyl ester

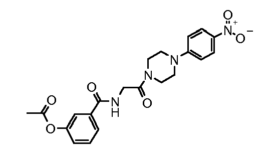

To a solution of 3.43 g (14.5 mmol) of (3-Acetoxy-benzoylamino)-acetic acid (3b) and 2.0 g (9.65 mmol) of 1-(4-Nitrophenyl)piperazine (Aldrich) in 100 mL DMF are added 5.0 g (9.65 mmol) PyBOP ((Benzotriazol-1-yloxy)tripyrrolidinophosphonium hexafluorophosphate) and 5 mL N-ethyl-N,N-diisopropylamine and the reaction mixture is stirred overnight at room temperature. After evaporation of the solvents the residue is chromatographed on silica gel using an ethyl acetate/ethanol gradient.
Yield: 1.2 g (29.2 %).

| Elemental analysis: | | | |
|---|---|---|---|
| calcd.: | C 59.15 | H 5.20 | N 13.14 |
| found: | C 59.38 | H 5.01 | N 13.25 |

### b) Acetic acid 3-{2-[4-(4-amino-phenyl)-piperazin-1-yl]-2-oxo-ethylcarbamoyl}-phenyl ester

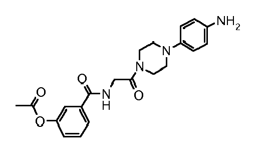

To a solution of 853 mg (2 mmol) of nitro compound 4a in 50 mL methanol/dichloromethane (1:1) is added a catalytic amount of Pd on charcoal (10%) and the suspension is stirred under hydrogen overnight at room temperature. The catalyst is filtered off and the solvent evaporated.
Yield: 795 mg (quantitative). The product is used in the next step without further purification.

### c) Acetic acid 3-{2-[4-(4-benzoylamino-phenyl)-piperazin-1-yl]-2-oxo-ethylcarbamoyl}-phenyl ester

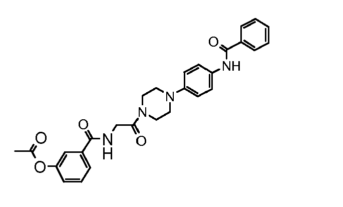

To a solution of 396 mg (1 mmol) acetic acid 3-{2-[4-(4-amino-phenyl)-piperazin-1-yl]-2-oxo-ethylcarbamoyl}-phenyl ester (4b) and 134 mg (1.1 mmol) of benzoic acid in 20 mL DMF are added 590 mg (1.14 mmol) of PyBOP and 0.35 mL (2 mmol) of N-ethyl-N,N-diisopropylamine and the reaction mixture is stirred at room temperature. After 4h the solvents are evaporated and the residue is chromatographed on silica gel using an dichloromethane/hexane gradient.
Yield: 0.31 g (62.1 %).

| Elemental analysis: | | | |
|---|---|---|---|
| calcd.: | C 67.19 | H 5.64 | N 11.19 |
| found: | C 66.94 | H 5.88 | N 11.02 |

### d) N-{2-[4-(4-Benzoylamino-phenyl)-piperazin-1-yl]-2-oxo-ethyl}-3-hydroxy-benzamide

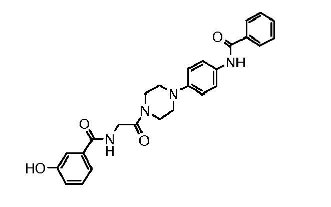

235 mg (0.47 mmol) of the acetate 4c are solved in 30 mL of ethanol and cooled to 0°C. After addition of 1.5 mL 3N NaOH the solution is stirred for 1 h, glacial acetic acid is added until the pH is below pH 7 and the solvents are evaporated. The raw product is crystallized from ethanol.
Yield: 177 mg (82 %).

| Elemental analysis: | | | |
|---|---|---|---|
| calcd.: | C 68.11 | H 5.72 | N 12.22 |
| found: | C 67.91 | H 5.60 | N 12.00 |

### e) Toluene-4-sulfonic acid 2-(3-{2-[4-(4-benzoylamino-phenyl)-piperazin-1-yl]-2-oxo-ethylcarbamoyl}-phenoxy)-ethyl ester

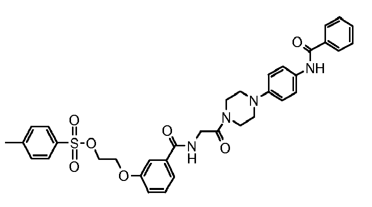

To a solution of 83 mg (0.18 mmol) of N-{2-[4-(4-Benzoylamino-phenyl)-piperazin-1-yl]-2-oxo-ethyl}-3-hydroxy-benzamide (4d) in 10 mL DMF are added 62 mg (0.45 mmol) of potassium carbonate and 100 mg (0.27 mmol) of 1,2 bis(tosyloxy)ethane (Aldrich) . The mixture is heated to 60°C for 3 h. After stirring overnight at room temperature the solvent is evaporated and the residue is chromatographed on silica gel using an ethyl acetate/hexane gradient.
Yield: 60 mg (51 %).

The compound has a purity >95% according to HPLC and is suitable as a precursor of the F-18 labelling.

### f) N-{2-[4-(4-Benzoylamino-phenyl)-piperazin-1-yl]-2-oxo-ethyl}-3-(2-[F-18]fluoro-ethoxy)-benzamide

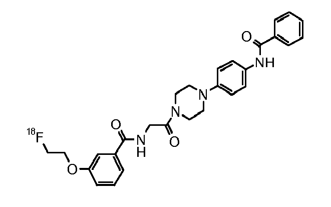

The tosylate precursor 4e was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.

The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.1 min. RCY (d.c.): 32 %. RCP: 99 %.

### g) N-{2-[4-(4-Benzoylamino-phenyl)-piperazin-1-yl]-2-oxo-ethyl}-3-(2-fluoro-ethoxy)-benzamide (HPLC standard)

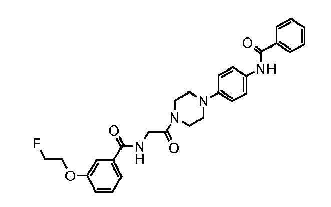

To a solution of 50 mg (0.11 mmol) of N-{2-[4-(4-Benzoylamino-phenyl)-piperazin-1-yl]-2-oxo-ethyl}-3-hydroxy-benzamide (4d) in 5 mL DMF are added 34 mg (0.25 mmol) of potassium carbonate and 10 uL (0.13 mmol) of 1-fluoro-2-bromoethane (ABCR, Germany). The mixture is heated to 60°C for 3 h. The solvent is evaporated and the residue is chromatographed on silica gel using an ethyl acetate/hexane gradient.
Yield: 34 mg (62 %).

| Elemental analysis: | | | | |
|---|---|---|---|---|
| calcd.: | C 66.65 | H 5.79 | F 3.77 | N 11.10 |
| found: | C 66.43 | H 5.89 | F 3.41 | N 11.31 |

### Example 5

### a) Toluene-4-sulfonic acid 2-{2-[2-(3-{2-[4-(4-benzyloxy-phenyl)-piperazin-1-yl]-2-oxo-ethylcarbamoyl}-phenoxy)-ethoxy]-ethoxy}-ethyl ester

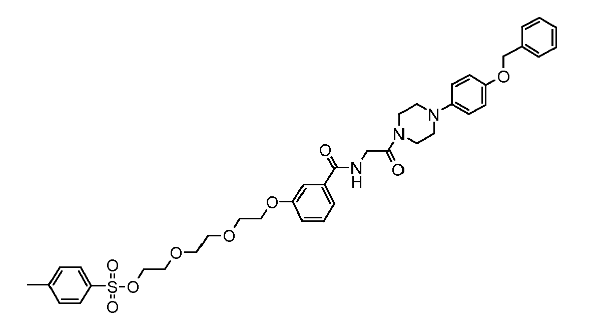

To a solution of 80 mg (0.18 mmol) of N-{2-[4-(4-Benzyloxy-phenyl)-piperazin-1-yl]-2-oxoethyl}-3-hydroxy-benzamide (3e) in 10 mL DMF are added 62 mg (0.45 mmol) of potassium carbonate and 124 mg (0.27 mmol) of tri(ethylene glycol) di-p-toluenesulfonate (Aldrich). The mixture is heated to 60°C for 3 h. After stirring overnight at room temperature the solvent is evaporated and the residue is chromatographed on silica gel using an ethyl acetate/hexane gradient.
Yield: 54 mg (41 %).

The compound has a purity >95% according to HPLC and is suitable as a precursor of the F-18 labelling.

### b) N-{2-[4-(4-Benzyloxy-phenyl)-piperazin-1-yl]-2-oxo-ethyl}-3-{2-[2-(2-[F-18]fluoro-ethoxy)-ethoxy]-ethoxy}-benzamide

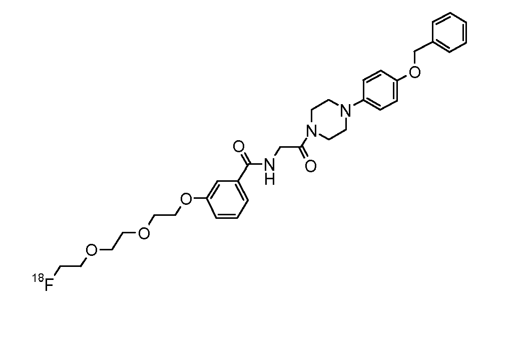

The tosylate precursor 5a was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.2 min. RCY (d.c.): 42 %. RCP: 99 %.

### c) N-{2-[4-(4-Benzyloxy-phenyl)-piperazin-1-yl]-2-oxo-ethyl}-3-{2-[2-(2-fluoro-ethoxy)-ethoxy]-ethoxy}-benzamide (HPLC standard)

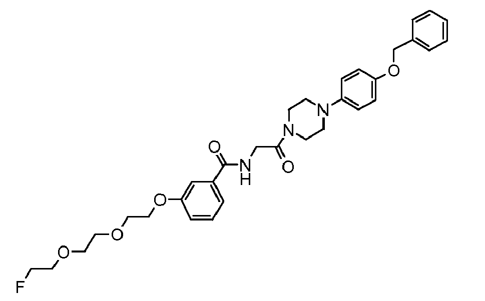

73 mg (0.10 mmol) of the tosylate precursor 5a are dissolved in 2mL acetonitrile. 6.8 mg (0.12 mmol) potassium fluoride and 44.4 mg Kryptofix in 1 mL acetonitrile are added and incubated for 10 min at 100°C. The reaction is checked by analytical HPLC. After completion of the reaction, the mixture is evaporated and the residue is chromatographed on silica gel using an ethyl acetate/hexane gradient.
Yield: 28 mg (49 %).

| Elemental analysis: | | | | |
|---|---|---|---|---|
| calcd.: | C 66.31 | H 6.61 | F 3.28 | N 7.25 |
| found: | C 66.07 | H 6.73 | F 3.11 | N 7.38 |

### Example 6

### a) Acetic acid 4-{4-[4-(4-nitrophenyl)-piperazin-1-yl]-4-oxo-butyryl}-phenyl ester

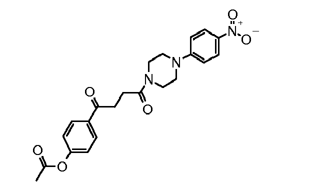

To a solution of 1.18 g (5 mmol) of 4-(4-acetoxyphenyl)-4-oxobutyric acid (Arch. Pharm. (Weinheim, Germany) 284; 292 (1951)) and 1.04 g (5 mmol) of 1-(4-nitrophenyl)piperazine (Aldrich) in 20 mL DMF are added 1.71 mL (10 mmol) of N-ethyl-N,N-diisopropylamine and 1.9 g (5 mmol) of 2-(1H-benzotriazol-1-yl)-tetramethyluronium hexafluorophosphate (HBTU). The reaction mixture is stirred at room temperature for 4h, then the solvents are evaporated and the residue is chromatographed on silica gel using an ethyl acetate/ethanol gradient.
Yield: 1.53 g (72 %).

| Elemental analysis: | | | |
|---|---|---|---|
| calcd.: | C 62.11 | H 5.45 | N 9.88 |
| found: | C 61.89 | H 5.54 | N 9.61 |

### b) Acetic acid 4-{4-[4-(4-amino-phenyl)-piperazin-1-yl]-4-oxo-butyryl}-phenyl ester

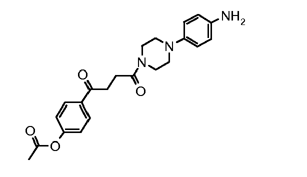

To a solution of 851 mg (2 mmol) of nitro compound 6a in 50 mL methanol/DMF (3:1) is added a catalytic amount of Pd on charcoal (10%) and the suspension is stirred under hydrogen overnight at room temperature. The catalyst is filtered off and the solvent evaporated. Yield: 791 mg (quantitative). The product is used in the next step without further purification.

### c) Acetic acid 4-[4-(4-{4-[(naphthalene-2-carbonyl)-amino]-phenyl}-piperazin-1-yl)-4-oxo-butyryl]-phenyl ester

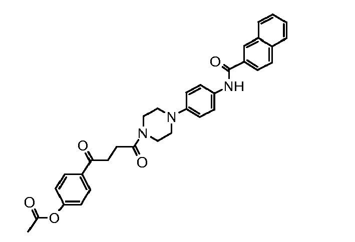

To a solution of 395 mg (1 mmol) acetic acid 4-{4-[4-(4-amino-phenyl)-piperazin-1-yl]-4-oxo-butyryl}-phenyl ester (6b) and 190 mg (1.1 mmol) of 2-naphthalene carboxylic acid (Aldrich) in 20 mL DMF are added 590 mg (1.14 mmol) of PyBOP and 0.35 mL (2 mmol) of N-ethyl-N,N-diisopropylamine and the reaction mixture is stirred at room temperature. After 4h the solvents are evaporated and the residue is chromatographed on silica gel using an dichloromethane/hexane gradient.
Yield: 374 mg (68 %).

| Elemental analysis: | | | |
|---|---|---|---|
| calcd.: | C 72.12 | H 5.69 | N 7.65 |
| found: | C 72.30 | H 5.61 | N 7.42 |

### d) Naphthalene-2-carboxylic acid (4-{4-[4-(4-hydroxy-phenyl)-4-oxo-butyryl]-piperazin-1-yl}-phenyl)-amide

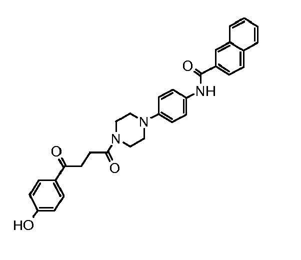

242 mg (0.44 mmol) of the acetate 6c are solved in 30 mL of ethanol/DMF (1:1) and cooled to 0°C. After addition of 2.2 mL 2N NaOH the solution is stirred for 30 min, glacial acetic acid is then added until the pH is below pH 7 and the solvents are evaporated. The raw product is crystallized from water and dried at 40°C in vacuo.
Yield: 223 mg (quantitative).

| Elemental analysis: | | | |
|---|---|---|---|
| calcd.: | C 73.35 | H 5.76 | N 8.28 |
| found: | C 73.10 | H 5.91 | N 8.25 |

### e) Toluene-4-sulfonic acid 2-{4-[4-(4-{4-[(naphthalene-2-carbonyl)-amino]-phenyl}-piperazin-1-yl)-4-oxo-butyryl]-phenoxy}-ethyl ester

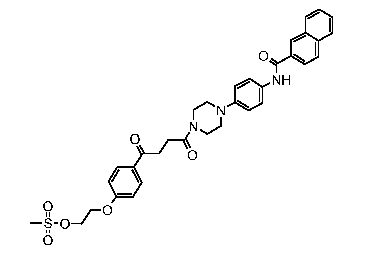

To a solution of 91 mg (0.18 mmol) of naphthalene-2-carboxylic acid (4-{4-[4-(4-hydroxyphenyl)-4-oxo-butyryl]-piperazin-1-yl}-phenyl)-amide (6d) in 10 mL DMF are added 62 mg (0.45 mmol) of potassium carbonate and 100 mg (0.27 mmol) of 1,2 bis(tosyloxy)ethane (Aldrich). The mixture is heated to 60°C for 3 h. After stirring overnight at room temperature the solvent is evaporated and the residue is chromatographed on silica gel using an ethyl acetate/hexane gradient.
Yield: 57 mg (46 %).

The compound has a purity >95% according to HPLC and is suitable as a precursor of the F-18 labelling.

### f) Naphthalene-2-carboxylic acid [4-(4-{4-[4-(2-[F-18]fluoro-ethoxy)-phenyl]-4-oxo-butyryl}-piperazin-1-yl)-phenyl]-amide

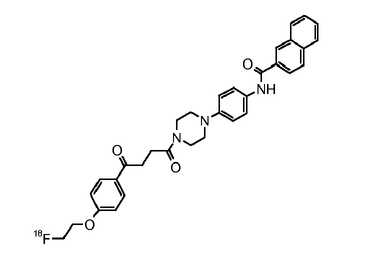

The tosylate precursor 6e was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 5.6 min. RCY (d.c.): 35 %. RCP: 98 %.

### Example 7

### a) Acetic acid 4-[4-(4-{4-[(naphthalene-l-carbonyl)-amino]-phenyl}-piperazin-1-yl)-4-oxo-butyryl]-phenyl ester

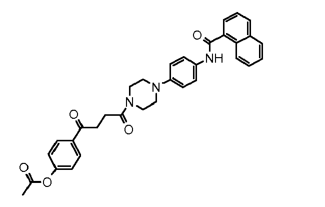

To a solution of 395 mg (1 mmol) acetic acid 4-{4-[4-(4-amino-phenyl)-piperazin-1-yl]-4-oxo-butyryl}-phenyl ester (6b) and 190 mg (1.1 mmol) of 1-naphthalene carboxylic acid (Aldrich) in 20 mL DMF are added 590 mg (1.14 mmol) of PyBOP and 0.35 mL (2 mmol) of N-ethyl-N,N-diisopropylamine and the reaction mixture is stirred at room temperature. After 4h the solvents are evaporated and the residue is chromatographed on silica gel using an dichloromethane/hexane gradient.
Yield: 336 mg (61 %).

| Elemental analysis: | | | |
|---|---|---|---|
| calcd.: | C 72.12 | H 5.69 | N 7.65 |
| found: | C 71.94 | H 5.77 | N 7.59 |

### b) Naphthalene-1-carboxylic acid (4-{4-[4-(4-hydroxy-phenyl)-4-oxo-butyryl]-piperazin-1-yl}-phenyl)-amide

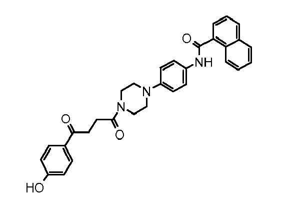

242 mg (0.44 mmol) of the acetate 7a are solved in 30 mL of ethanol/DMF (1:1) and cooled to 0°C. After addition of 2.2 mL 2N NaOH the solution is stirred for 30 min, glacial acetic acid is then added until the pH is below pH 7 and the solvents are evaporated. The raw product is crystallized from water and dried at 40°C in vacuo.
Yield: 223 mg (quantitative).

| Elemental analysis: | | | |
|---|---|---|---|
| calcd.: | C 73.35 | H 5.76 | N 8.28 |
| found: | C 73.21 | H 5.62 | N 8.11 |

### c) Toluene-4-sulfonic acid 2-{4-[4-(4-{4-[(naphthalene-1-carbonyl)-amino]-phenyl}-piperazin-1-yl)-4-oxo-butyryl]-phenoxy}-ethyl ester

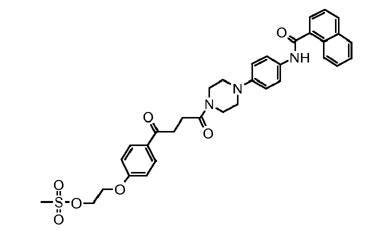

To a solution of 91 mg (0.18 mmol) of naphthalene-1-carboxylic acid (4-{4-[4-(4-hydroxyphenyl)-4-oxo-butyryl]-piperazin-1-yl}-phenyl)-amide (7b) in 10 mL DMF are added 62 mg (0.45 mmol) of potassium carbonate and 100 mg (0.27 mmol) of 1,2 bis(tosyloxy)ethane (Aldrich). The mixture is heated to 60°C for 3 h. After stirring overnight at room temperature the solvent is evaporated and the residue is chromatographed on silica gel using an ethyl acetate/hexane gradient.
Yield: 63 mg (51 %).

The compound has a purity >95% according to HPLC and is suitable as a precursor of the F-18 labelling.

### d) Naphthalene-1-carboxylic acid [4-(4-{4-[4-(2-[F-18]fluoro-ethoxy)-phenyl]-4-oxo-butyryl}-piperazin-1-yl)-phenyl]-amide

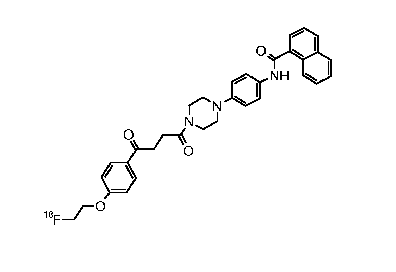

The tosylate precursor 7c was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 6.0 min. RCY (d.c.): 32 %. RCP: 97 %.

### Example 8

### a) Acetic acid 4-{4-[4-(4-benzyloxy-phenyl)-piperazin-1-yl]-4-oxobutyryl}-phenyl ester

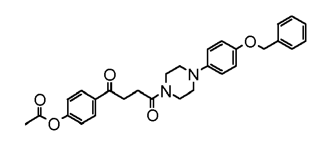

To a solution of 652 mg (2.76 mmol) of 4-(4-acetoxyphenyl)-4-oxobutyric acid (Arch. Pharm. (Weinheim, Germany) 284; 292 (1951)) in 70 mL THF and 0.40 mL triethyl amine (2.87 mmol) at -15°C, 0.396 mL (3.03 mmol) isobutyl chloroformate are added dropwise and the solution is maintained at this temperature for another 15 min. Then, 740 mg of 1-(4-benzyloxy-phenyl)-piperazine 3c and 1.7 mL triethyl amine (12.25 mmol) in 30 mL THF and 30 mL dichloromethane are added slowly to this cold solution, the temperature is kept below 10°C for another 15 min and is then allowed to reach room temperature. After stirring overnight the solvent is evaporated and the residue is chromatographed on silica gel using a hexane/ethyl acetate gradient.
Yield: 219 mg (45 %).

| Elemental analysis: | | | |
|---|---|---|---|
| calcd.: | C 71.59 | H 6.21 | N 5.76 |
| found: | C 71.33 | H 6.46 | N 5.60 |

### b) 1-[4-(4-Benzyloxy-phenyl)-piperazin-1-yl]-4-(4-hydroxyphenyl)-butane-1,4-dione

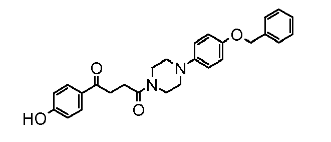

229 mg (0.47 mmol) of the acetate 8a are solved in 30 mL of ethanol and cooled to 0°C. After addition of 1.5 mL 3N NaOH the solution is stirred for 1 h, glacial acetic acid is added until the pH is below pH 7 and the solvents are evaporated. The raw product is crystallized from ethanol.
Yield: 186 mg (89 %).

| Elemental analysis: | | | |
|---|---|---|---|
| calcd.: | C 72.95 | H 6.35 | N 6.30 |
| found: | C 72.78 | H 6.27 | N 6.41 |

### c) Toluene-4-sulfonic acid 2-(4-{4-[4-(4-benzyloxy-phenyl)-piperazin-1-yl]-4-oxobutyryl}-phenoxy)-ethyl ester

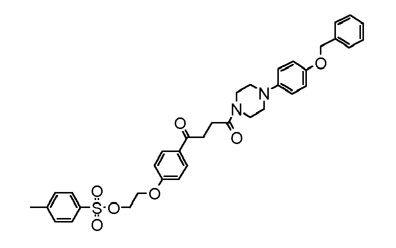

To a solution of 80 mg (0.18 mmol) of 1-[4-(4-Benzyloxy-phenyl)-piperazin-1-yl]-4-(4-hydroxyphenyl)-butane-1,4-dione (8b) in 10 mL DMF are added 62 mg (0.45 mmol) of potassium carbonate and 100 mg (0.27 mmol) of 1,2 bis(tosyloxy)ethane (Aldrich) . The mixture is heated to 60°C for 3 h. After stirring overnight at room temperature the solvent is evaporated and the residue is chromatographed on silica gel using an ethyl acetate/hexane gradient.
Yield: 60 mg (52 %).

The compound has a purity >95% according to HPLC and is suitable as a precursor of the F-18 labelling.

### d) 1-[4-(4-Benzyloxy-phenyl)-piperazin-1-yl]-4-[4-(2-[F-18]fluoro-ethoxy)-phenyl]-butane-1,4-dione

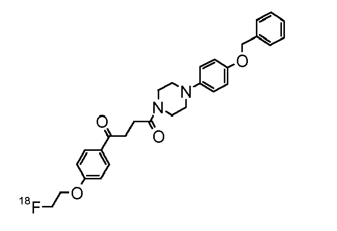

The tosylate precursor 8c was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.7 min. RCY (d.c.): 45 %. RCP: 99 %.

### Example 9

### a) Acetic acid 3-[2-(4-{4-[(naphthalene-2-carbonyl)-amino]-phenyl}-piperazin-1-yl)-2-oxo-ethylcarbamoyl]-phenyl ester

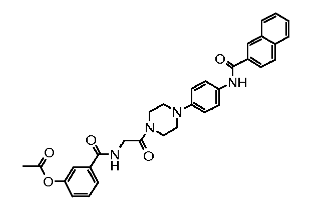

To a solution of 396 mg (1 mmol) acetic acid 3-{2-[4-(4-amino-phenyl)-piperazin-1-yl]-2-oxo-ethylcarbamoyl}-phenyl ester (4b) and 190 mg (1.1 mmol) of 2-naphthalene carboxylic acid (Aldrich) in 20 mL DMF are added 590 mg (1.14 mmol) of PyBOP and 0.35 mL (2 mmol) of N-ethyl-N,N-diisopropylamine and the reaction mixture is stirred at room temperature. After 4h the solvents are evaporated and the residue is chromatographed on silica gel using an dichloromethane/hexane gradient.
Yield: 0.25 g (43.9 %).

| Elemental analysis: | | | |
|---|---|---|---|
| calcd.: | C 69.80 | H 5.49 | N 10.18 |
| found: | C 69.55 | H 5.61 | N 10.03 |

### b) Naphthalene-2-carboxylic acid (4-{4-[2-(3-hydroxy-benzoylamino)-acetyl]-piperazin-1-yl}-phenyl)-amide

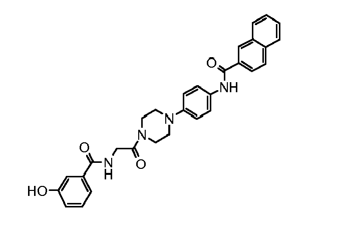

240 mg (0.44 mmol) of the acetate 9a are solved in 30 mL of ethanol/DMF (1:1) and cooled to 0°C. After addition of 2.2 mL 2N NaOH the solution is stirred for 30 min, glacial acetic acid is then added until the pH is below pH 7 and the solvents are evaporated. The raw product is crystallized from water and dried at 40°C *in vacuo*.
Yield: 220 mg (quantitative).

| Elemental analysis: | | | |
|---|---|---|---|
| calcd.: | C 70.85 | H 5.55 | N 11.02 |
| found: | C 70.53 | H 5.77 | N 10.85 |

### c) Toluene-4-sulfonic acid 2-{3-[2-(4-{4-[(naphthalene-2-carbonyl)-amino]-phenyl}-piperazin-1-yl)-2-oxo-ethylcarbamoyl]-phenoxy}-ethyl ester

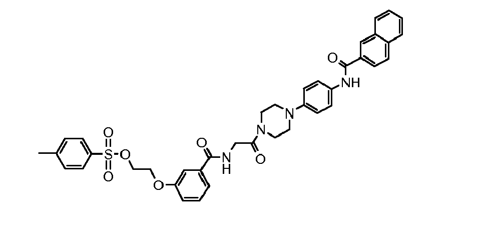

To a solution of 92 mg (0.18 mmol) of Naphthalene-2-carboxylic acid (4-{4-[2-(3-hydroxy-benzoylamino)-acetyl]-piperazin-1-yl}-phenyl)-amide (9b) in 10 mL DMF are added 62 mg (0.45 mmol) of potassium carbonate and 100 mg (0.27 mmol) of 1,2 bis(tosyloxy)ethane (Aldrich). The mixture is heated to 60°C for 3 h. After stirring overnight at room temperature the solvent is evaporated and the residue is chromatographed on silica gel using an ethyl acetate/hexane gradient.
Yield: 58 mg (46 %).

The compound has a purity >95% according to HPLC and is suitable as a precursor of the F-18 labelling.

### d) Naphthalene-2-carboxylic acid [4-(4-{2-[3-(2-[F-18]fluoro-ethoxy)-benzoylamino]-acetyl}-piperazin-1-yl)-phenyl]-amide

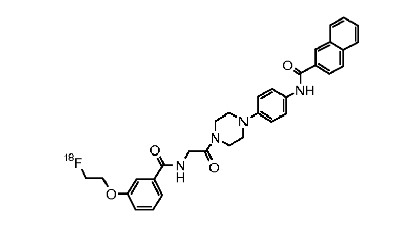

The tosylate precursor 9c was dissolved in dry dimethylformamide (100 µL) and acetonitrile (400 µL) and added to the dry potassium/ Kryptofix/ fluoride complex (vide supra). The solution was heated to 110 °C for 6 min and then allowed to cool to room temperature for 5 min. The solution was diluted with water to give a final volume of 10 mL and was passed through a Sep-Pak Plus C18 cartridge (Waters, WAT020515). The cartridge was rinsed with water (20 ml) and the compound was eluted with acetonitrile (3 mL). The eluted compound was diluted with water to yield 5 mL and was subjected to preparative HPLC purification. A Gemini 5 µ C 18 110 Å, 250 * 10 mm (Phenomenex, 00G-4435-N0) was used as a stationary phase. A water (A)/ acetonitrile (B) 3 / 7 mixture was used as a mobile phase with a flow rate of 3 mL/ min.
The solution was diluted with water to give a final volume of 20 mL and was passed through a Sep-Pak light C18 cartridge (Waters, WAT023501). The cartridge was rinsed with water (10 ml) and the compound was eluted with ethanol (0.5 mL). The final product was characterized by HPLC on a Gemini 5 µ C 18 110 Å, 250 * 4,6 mm (Phenomenex, 00G-4435-E0) HPLC column with an isocratic water/ acetonitrile (2/8; v/v) mixture at a flow rate of 1 mL/ min. Retention time (t_{R}): 4.6 min. RCY (d.c.): 39 %. RCP: 99 %.

### e) Naphthalene-2-carboxylic acid [4-(4-{2-[3-(2-fluoro-ethoxy)-benzoylamino]-acetyl}-piperazin-1-yl)-phenyl]-amide (HPLC-Standard)

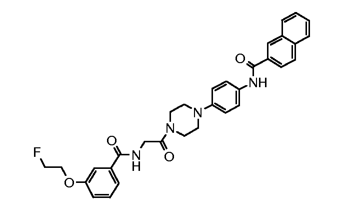

To a solution of 56 mg (0.11 mmol) of Naphthalene-2-carboxylic acid (4-{4-[2-(3-hydroxy-benzoylamino)-acetyl]-piperazin-1-yl}-phenyl)-amide (9b) in 5 mL DMF are added 34 mg (0.25 mmol) of potassium carbonate and 10 uL (0.13 mmol) of 1-fluoro-2-bromoethane (ABCR, Germany). The mixture is heated to 60°C for 3 h. The solvent is evaporated and the residue taken up in dimethyl sulfoxide and chromatographed twice on RP-18 using a water/acetonitrile gradient.
Yield: 24 mg (40 %).

| Elemental analysis: | | | | |
|---|---|---|---|---|
| calcd.: | C 69.30 | H 5.63 | F 3.43 | N 10.10 |
| found: | C 69.15 | H 5.81 | F 3.22 | N 10.28 |

### Example 10

### a) {2-[4-(4-Benzyloxy-phenyl)-piperazin-1-yl]-2-oxo-ethyl}-carbamic acid tert-butyl ester

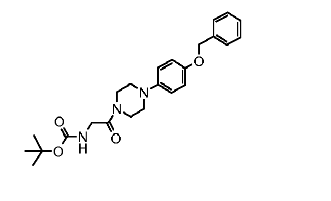

To a solution of 7.7 g (43.97 mmol) t-Butoxycarbonyl-glycine (Aldrich) in 800 mL THF and 8 mL triethyl amine (57.71 mmol) at -15°C, 5.75 mL (43.97 mmol) isobutyl chloroformate are added dropwise and the solution is maintained at this temperature for another 15 min. Then, 11.8 g of 1-(4-benzyloxyphenyl)-piperazine (3c) and 29 mL triethyl amine (210 mmol) in 300 mL THF/dichloromethane (1:1) are added slowly to this cold solution, the temperature is kept below 10°C for another 15 min and is then allowed to reach room temperature. After stirring overnight the solvent is evaporated and the residue is taken up in ethyl acetate. This solution is washed successively with aqueous sodium carbonate, water, 1 M aqueous HCl solution, saturated aqueous sodium chloride solution, finally dried over magnesium sulfate and then evaporated. This residue is chromatographed on silica gel using a hexane/ethyl acetate gradient.
Yield: 15.51 g (82.9 %).

| Elemental analysis: | | | |
|---|---|---|---|
| calcd.: | C 67.74 | H 7.34 | N 9.87 |
| found: | C 67.33 | H 7.47 | N 9.62 |

### b) 2-[4-(4-Benzyloxy-phenyl)-piperazin-1-yl]-2-oxo-ethyl-ammonium chloride

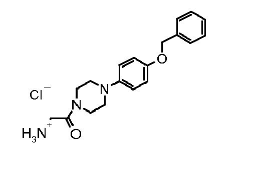

17.0 g (39.95 mmol) of {2-[4-(4-Benzyloxy-phenyl)-piperazin-1-yl]-2-oxo-ethyl}-carbamic acid tert-butyl ester (10a) are suspended in 300 mL 2N HCl in diethyl ether and stirred overnight at room temperature. The precipitate is filtered off and washed with ether and dried at 40°C *in vacuo*.
Yield: 14.5 g (quantitative). The product is used in the next step without further purification.

### c) N-{2-[4-(4-Benzyloxy-phenyl)-piperazin-1-yl]-2-oxo-ethyl}-6-bromo-nicotinamide

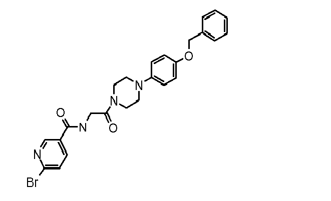

To a solution of 202 mg (1 mmol) of 2-bromo-5-pyridinecarboxylic acid (Aldrich) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient.
Yield: 183 mg (35.9 %).

| Elemental analysis: | | | | |
|---|---|---|---|---|
| calcd.: | C 58.95 | H 4.95 | Br 15.69 | N 11.00 |
| found: | C 58.66 | H 5.18 | Br 15.20 | N 11.36 |

The compound has a purity >95% according to HPLC and is suitable as a precursor for the F-18 labelling.

### d) N-{2-[4-(4-Benzyloxy-phenyl)-piperazin-1-yl]-2-oxo-ethyl}-6-fluoro-nicotinamide

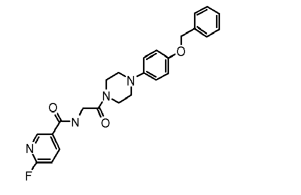

To a solution of 141 mg (1 mmol) of 2-fluoro-5-pyridinecarboxylic acid (Aldrich) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient.
Yield: 302 mg (67.3 %).

| Elemental analysis: | | | | |
|---|---|---|---|---|
| calcd.: | C 66.95 | H 5.62 | F 4.24 | N 12.49 |
| found: | C 66.84 | H 5.97 | F 3.95 | N 12.52 |

### e) N-{2-[4-(4-Benzyloxy-phenyl)-piperazin-1-yl]-2-oxo-ethyl}-6-[F-18]-fluoro-nicotinamide

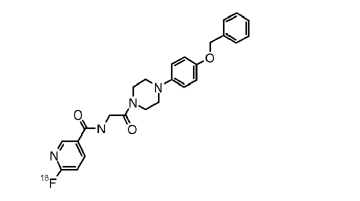

Aqueous [¹⁸F]Fluoride (5.1 GBq) was trapped on a QMA cartridge (Waters, Sep Pak Light QMA Part.No.: WAT023525 ) and eluted with 5mg K_{2.2.2} in 0,95ml MeCN +1 mg K₂CO₃ in 50µl water into a Wheaton vial (5ml). The solvent was removed by heating at 120°C for 10 min under a stream of nitrogen. Anhydrous MeCN (1 ml) was added and evaporated as before. A solution of precursor **10c** (5 mg) in 700 µl anhydrous DMSO was added. After heating at 180°C for 30 min the crude reaction mixture was diluted with water to a total volume and purified by preparative HPLC: ACE 5-C18-HL 250mmx10mm, Advanced Chromatography Technologies; Cat.No.: ACE 321-2510; isocratic, 35% acetonitrile in 0.1% trifluoroacetic acid, flow: 4 ml/min; t_{R}=18min. The collected HPLC fraction was diluted with 40ml water and immobilized on a Sep-Pak light C18 cartridge (Waters, WAT023501), which was washed with 5ml water and eluted with 1ml ethanol to deliver 1015 MBq of the product (36%, corrected for decay; radiochemical purity >99%). The desired product was characterized by co-injection with the non-radioactive F-19 fluoro standard on the analytical HPLC: Agilent ZORBAX 300SB-C18 250*4.6 mm ; 5 µm Agilent; PN 880995-902; A): Water + 0,1% TFA, B): MeCN + 0,1% TFA, 0 to 10 min, 35% B; 10 to 10:30 min, 35% B to 100%B; 1mL/min (t_{R}=7.6min), RCP: 99 %(HPLC).

### Example 11

### a) N-{2-[4-(4-Benzyloxy-phenyl)-piperazin-1-yl]-2-oxo-ethyl}-2-bromo-isonicotinamide

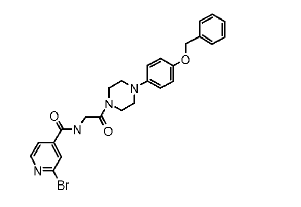

To a solution of 202 mg (1 mmol) of 2-bromo-4-pyridinecarboxylic acid (Alfa) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient. The product containing fractions are collected and recrystallized from ethyl acetate.
Yield: 165 mg (32.4 %).

| Elemental analysis: | | | | |
|---|---|---|---|---|
| calcd.: | C 58.95 | H 4.95 | Br 15.69 | N 11.00 |
| found: | C 58.32 | H 5.09 | Br 15.11 | N 10.73 |

The compound has a purity >95% according to HPLC and is suitable as a precursor for the F-18 labelling.

### b) N-{2-[4-(4-Benzyloxy-phenyl)-piperazin-1-yl]-2-oxo-ethyl}-2-fluoro-isonicotinamide

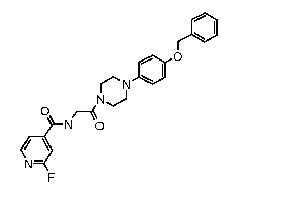

To a solution of 141 mg (1 mmol) of 2-fluoro-4-pyridinecarboxylic acid (Aldrich) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient.
Yield: 326 mg (72.7 %).

| Elemental analysis: | | | | |
|---|---|---|---|---|
| calcd.: | C 66.95 | H 5.62 | F 4.24 | N 12.49 |
| found: | C 66.58 | H 5.81 | F 4.03 | N 12.68 |

### c) N-{2-[4-(4-Benzyloxy-phenyl)-piperazin-1-yl]-2-oxo-ethyl}-2-[F-18]-fluoro-isonicotinamide

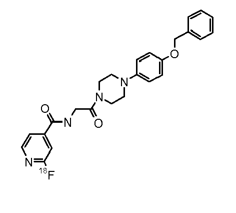

Aqueous [¹⁸F]Fluoride (4,9 GBq) was trapped on a QMA cartridge (Waters, Sep Pak Light QMA Part.No.: WAT023525 ) and eluted with 5mg K_{2.2.2} in 0,95ml MeCN +1 mg K₂CO₃ in 50µl water into a Wheaton vial (5ml). The solvent was removed by heating at 120°C for 10 min under a stream of nitrogen. Anhydrous MeCN (1 ml) was added and evaporated as before. A solution of precursor **11a** (5 mg) in 700 µl anhydrous DMSO was added. After heating at 180°C for 30 min the crude reaction mixture was diluted with water to a total volume of 5mL and purified by preparative HPLC: ACE 5-C18-HL 250mmx10mm, Advanced Chromatography Technologies; Cat.No.: ACE 321-2510; isocratic, 35% acetonitrile in 0.1% trifluoroacetic acid, flow: 4 ml/min; t_{R}=18min. The collected HPLC fraction was diluted with 40ml water and immobilized on a Sep-Pak light C18 cartridge (Waters, WAT023501), which was washed with 5ml water and eluted with 1ml ethanol to deliver 1293 MBq of the product (44%, corrected for decay) which was characterized and reconfirmed by co-injection with the non-radioactive F-19 fluoro standard using analytical HPLC: Agilent ZORBAX 300SB-C18 50*4.6 mm ; 5 µm Agilent; PN 880995-902, A): Water + 0, 1 % TFA, B): MeCN + 0, 1 % TFA, 0 to 10 min, 35% B; 10 to 10:30 min, 35% B to 100%B; 1mL/min, (t_{R}=7.5min), RCP: >99 %(HPLC).

### Example 12

### a) N-{2-[4-(4-Benzyloxy-phenyl)-piperazin-1-yl]-2-oxo-ethyl}-2-bromo-nicotinamide

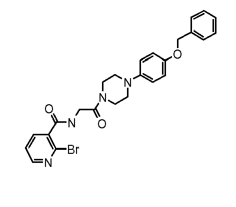

To a solution of 202 mg (1 mmol) of 2-bromo-nicotinic acid (Aldrich) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient.
Yield: 210 mg (41.2 %).

| Elemental analysis: | | | | |
|---|---|---|---|---|
| calcd.: | C 58.95 | H 4.95 | Br 15.69 | N 11.00 |
| found: | C 59.11 | H 4.75 | Br 15.39 | N 11.03 |

The compound has a purity >95% according to HPLC and is suitable as a precursor for the F-18 labelling.

### b) N-{2-[4-(4-Benzyloxy-phenyl)-piperazin-1-yl]-2-oxo-ethyl}-2-fluoro-nicotinamide

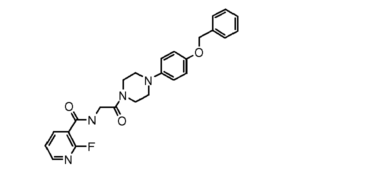

To a solution of 141 mg (1 mmol) of 2-fluoro-nicotinic acid (Aldrich) and 398 mg (1.1 mmol) of 2-[4-(4-benzyloxyphenyl)-piperazin-1-yl]-2-oxoethyl-ammonium chloride (10b) in 20 mL DMF are added 624 mg (1.2 mmol) PyBOP and 0.61 mL N-ethyl-N,N-diisopropylamine and the reaction mixture is stirred overnight at room temperature. After evaporation of the solvent the residue is chromatographed on silica gel using an ethyl acetate/ethanol gradient.
Yield: 326 mg (72.7 %).

| Elemental analysis: | | | | |
|---|---|---|---|---|
| calcd.: | C 66.95 | H 5.62 | F 4.24 | N 12.49 |
| found: | C 66.71 | H 5.79 | F 3.88 | N 12.30 |

### c) N-{2-[4-(4-Benzyloxy-phenyl)-piperazin-1-yl]-2-oxo-ethyl}-2-[F-18]-fluoro-nicotinamide

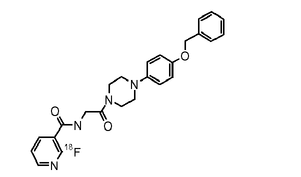

Aqueous [¹⁸F]Fluoride (7 GBq) was trapped on a QMA cartridge (Waters, Sep Pak Light QMA Part.No.: WAT023525 ) and eluted with 5mg K_{2.2.2} in 0,95ml MeCN +1 mg K₂CO₃ in 50µl water into a Wheaton vial (5ml). The solvent was removed by heating at 120°C for 10 min under a stream of nitrogen. Anhydrous MeCN (1 ml) was added and evaporated as before. A solution of precursor **12a** (5 mg) in 700 µl anhydrous DMSO was added. After heating at 180°C for 30 min the crude reaction mixture was diluted with water to a total volume of 5mL and purified by preparative HPLC: ACE 5-C18-HL 250mmx10mm, Advanced Chromatography Technologies; Cat.No.: ACE 321-2510; isocratic, 35% acetonitrile in 0.1% trifluoroacetic acid, flow: 4 ml/min; t_{R}=19min. The collected HPLC fraction was diluted with 40ml water and immobilized on a Sep-Pak light C18 cartridge (Waters, WAT023501), which was washed with 5ml water and eluted with 1ml ethanol to deliver 2015 MBq of the product (49%, corrected for decay) which was characterized and reconfirmed by co-injection with the non-radioactive F-19 fluoro standard using analytical HPLC: Agilent ZORBAX 300SB-C18 50*4.6 mm ; 5 µm Agilent; PN 880995-902, A): Water + 0, 1 % TFA, B): MeCN + 0, 1 % TFA, 0 to 10 min, 35% B; 10 to 10:30 min, 35% B to 100%B; 1mL/min, (t_{R}=7.8min), RCP: >99 %(HPLC).

### Example 13: Biological data

### a) Methods

### Binding studies using human brain homogenate

A competition assay with a tritiated amyloid ligand was performed in 96-well plates (Greiner bio-one; Cat. 651201; Lot. 06260130) using brain homogenate from AD patients.
Homogenates were prepared by homogenizing (Ultra-Turrax, setting 2, 30 s, 24000 rpm) dissected frontal cortex containing grey matter and white matter from AD patients in phosphate buffered saline (PBS, pH 7.4). The homogenate with a concentration of 100 mg wet tissue/ml was divided into aliquots of 300 µl and stored at -80°C.

Varying concentrations of the unlabeled test substances were incubated with 100 µg/ml homogenate and 10 nM of the tritiated ligand in PBS, 0.1% BSA (final volume 200 µl) for 3 h at room temperature. Subsequently the binding mixture was filtered through Whatman GF/B filters (wetted with PBS, 0.1% BSA) using a Filtermate 196 harvester (Packard). Filters were then washed twice with PBS, 0.1%BSA and 40 µl scintillator was added to each well before the bound radioactivity was measured in a TopCount devise (Perkin Elmer). Non-specific binding was assessed by adding an access of 1000x of the tritiated ligand to the reaction mixture. Finally IC50 values were calculated with the help of appropriate analysis software.

### Autoradiographical analysis

Fresh frozen as well as paraffin embedded sections of the frontal lobe from Alzheimer's dementia patients, frontotemporal dementia patients and age matched controls were used for the study.
Frozen sections, sliced at 18 µm thickness on a cryostate (Leica, Germany) and paraffin sections, sliced on a sliding microtom (Leica) at a thickness of 6 µm, were mounted onto glass slides (Superfrost Plus, Fa.Menzel, Braunschweig Germany). Frozen sections were allowed to adhere to the slides for several nights at -20°C. The paraffin sections were deparaffinized using routine histological methods. For binding studies sections were incubated with the F-18 labeled test compound at 10 Bq/µl diluted in 25mM Hepes buffer, pH 7.4, 0,1% (BSA) (200-300 µl/slide) for 1,5 hour at room temperature in a humidified chamber. For blocking experiments a 1000-fold access of the unlabeled test substance was added to the incubation mixture. After hybridization, sections were washed four times with Hepes buffer, 0.1% BSA (or alternatively two times with 40% ethanol) and finally dipped two times into dest. water for 10 sec. The air-dried sections were exposed to imaging plates and signals were detected by a phosphoimager device (Fuji BAS5000).

### Biodistribution

Biodistribution and excretion studies were performed in male NMRI mice (body weight app. 30 g; 3 animals per time point). The animals were kept under normal laboratory conditions at a temperature of 22 ± 2°C and a dark/light rhythm of 12 hours. Food and water were provided ad libitium. During an acclimation period of at least 3 days before the beginning of the study animals were clinically examined to ascertain the absence of abnormal clinical signs.
At 2, 5, 30, 60, 240 min post intravenous injection via the tail vein of ca. 150 kBq in 100 µl of the test compound, urine and feces were quantitatively collected. At the same time points, animals were sacrificed by decapitation and under isoflurane anaesthesia and the following organs and tissues were removed for the determination of radioactivity using a gamma-counter: spleen, liver, kidney, lung, femur, heart, brain, fat, thyroid, muscle, skin, blood, tail, stomach (without content), testicle, intestine (with content), pancreas, adrenals, and the remaining body. For analysis the decay corrected percentage of the injected dose per tissue weight (%ID/g ± standard deviation) was calculated.

### b) Results

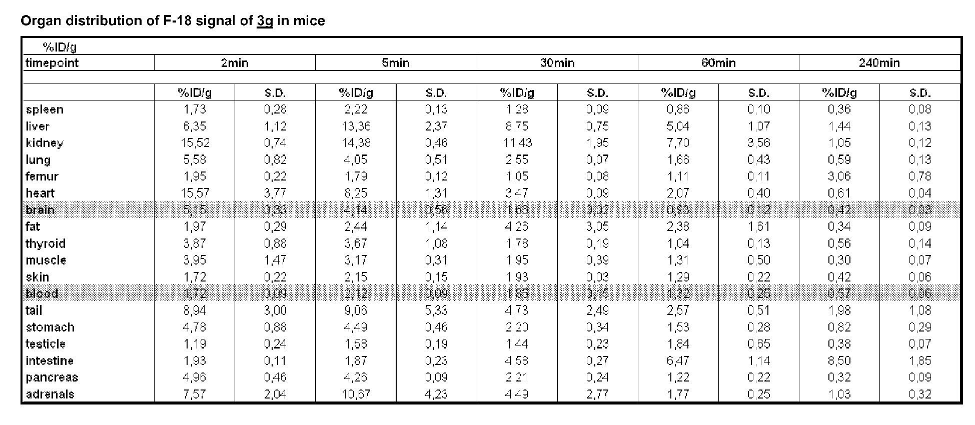

### Autoradiography

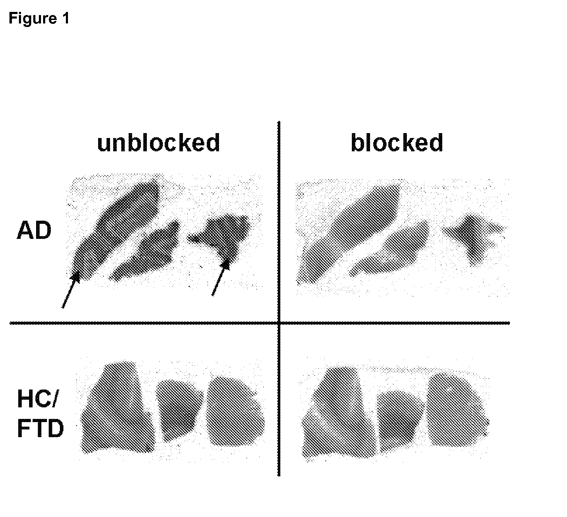

Fig. 1 shows the autoradiographical analysis of binding of 3g to cryosections from cortex of Alzheimer's disease patients (AD) and controls without Aβ plaques (HC/FTD) (healthy control/ frontotemporal dementia). Specific binding in plaque-rich regions of AD samples is indicated by arrows.

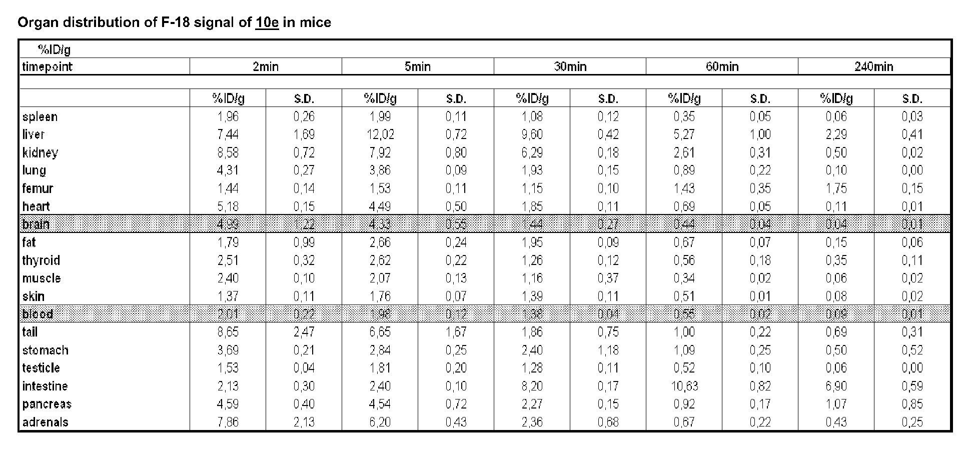

### Autoradiography

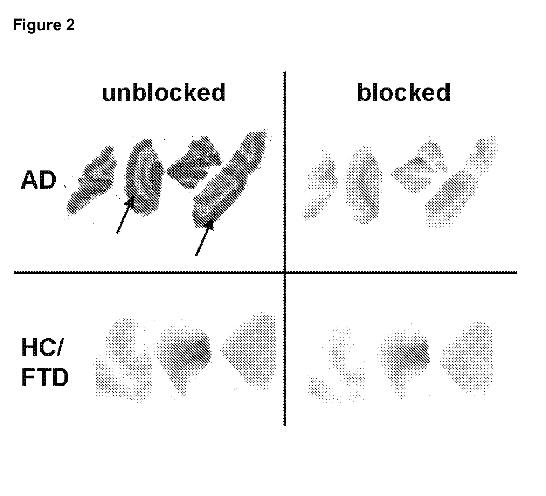

Fig. 2 shows the autoradiographical analysis of binding of example 10e to cryosections from cortex of Alzheimer's disease patients (AD) and controls without Aβ plaques (HC/FTD) (healthy control/ frontotemporal dementia). Specific binding in plaque-rich regions of AD samples is indicated by arrows.

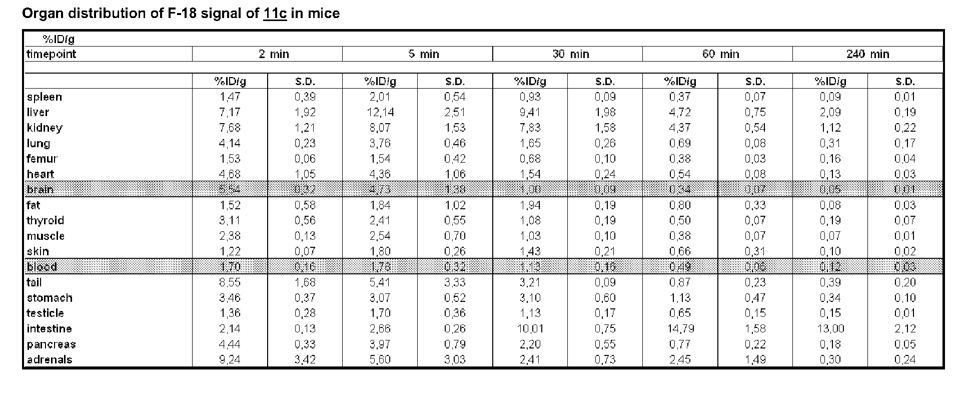

### Autoradiography

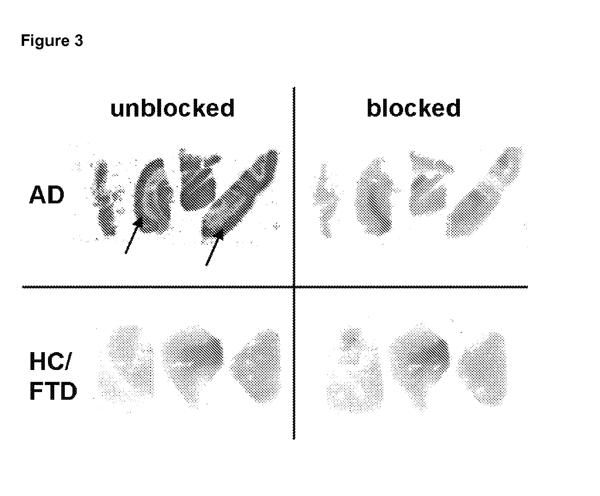

Fig. 3 shows the Autoradiographical analysis of binding of example 11c to cryosections from cortex of Alzheimer's disease patients (AD) and controls without Aβ plaques (HC/FTD) (healthy control/ frontotemporal dementia). Specific binding in plaque-rich regions of AD samples is indicated by arrows.

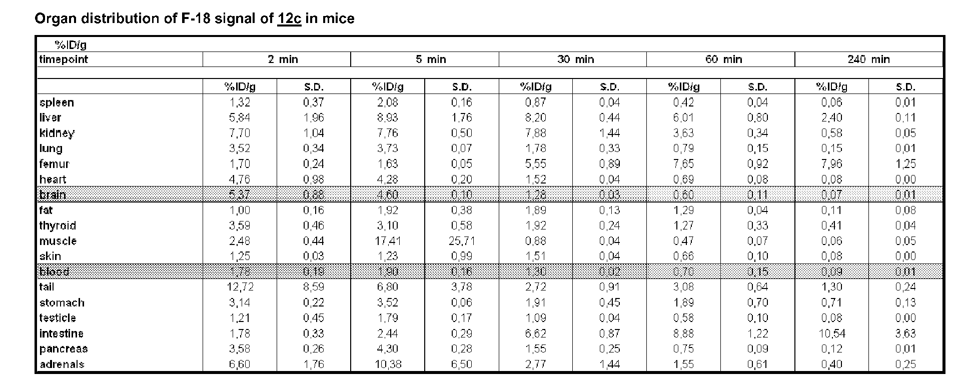

### Autoradiography

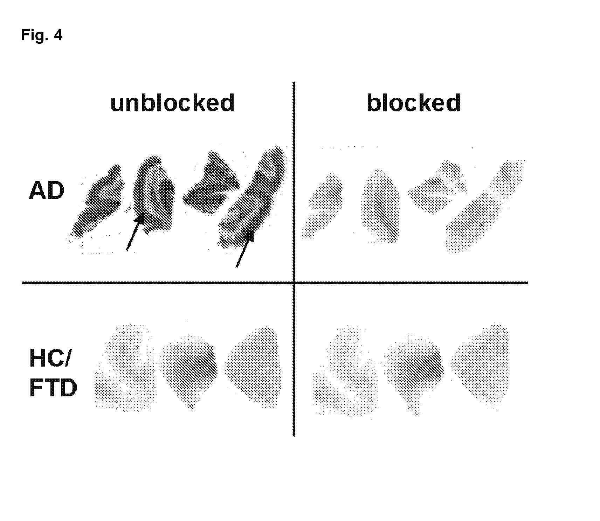

Fig. 4 shows the autoradiographical analysis of binding of example 12c to cryosections from cortex of Alzheimer's disease patients (AD) and controls without Aβ plaques (HC/FTD) (healthy control/ frontotemporal dementia). Specific binding in plaque-rich regions of AD samples is indicated by arrows.

### IC50 values of selected compounds

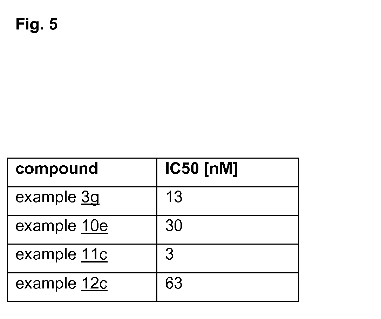

Fig. 5 shows IC50 values in [nM] of selected compounds measured in a competition assay using brain homogenate from AD patients.

## Claims

1. A compound of formula I 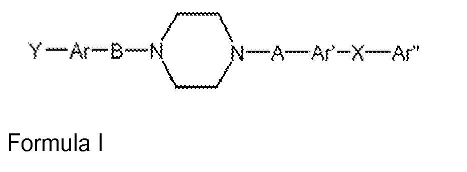 or a pharmaceutically acceptable salt or a prodrug thereof,
wherein
- Y is selected from the group consisting of: F, Cl, Br, I, H, ¹⁸F, ¹⁹F, ⁷⁶Br, ¹²³I, ¹²⁵I, ¹¹C, ³H, ¹³N, ¹⁵O; a leaving group, tosyl, brosyl, nosyl, triflate, sulfonate, substituted sulfonate, mesylate, and nonaflate; and if directly bound to an aromatic C-atom iodonium-aryl I⁺-aryl, trialkylammonium, preferred trimethylammonium, and NO₂;
- Ar is selected from the group consisting of:
substituted or non-substituted mono-, bi- or tricyclic aromatic or heteroaromatic ring systems;
- B is selected from the group consisting of:
direct bond, a branched or non-branched alkyl or alkylenchain comprised of 1-10 C-atoms;
- A is selected from the group consisting of:
a direct bond, and CO-NH, CS-NH;
- Ar' is selected from the group consisting of:
substituted or non-substitued mono-, or bi-cyclic aromatic or heteroaromatic ring systems;
- X is selected from the group consisting of:
a direct bond or a substituted or non-substituted C₁-C₃ alkyl chain;
- Ar" is selected from the group consisting of:
substituted or non-substituted mono-, or bi-cyclic aromatic or heteroaromatic ring systems.

2. A compound according to claim 1, wherein
- the heteroaromatic or aromatic ring systems of Ar are optionally substituted by one or two alkyl, alkylen, alkynesubstituents and/or alkoxysubstituents, and wherein
- the mono-, bi- or tricyclic aromatic or heteroaromatic ring systems may be further substituted by electron withdrawing groups directly bound to an aromatic C-atom; and wherein
- the alkylen chain of B may comprise 1 or 2 unsaturated bondings, and wherein the alkyl or alkylenchain is optionally interrupted by N, S, SO, SO₂ or O, and wherein the alkyl or alkylenchain is optionally substituted by oxo or -OH; and wherein
- the heteroaromatic or aromatic ring systems of Ar' are optionally substituted by one or two alkyl, alkylen, alkynesubstituents and/or alkoxysubstituents and wherein the mono-, bi- or tricyclic aromatic or heteroaromatic ring systems may be further substituted by electron withdrawing groups directly bound to an aromatic C-atom; and wherein
- the C₁-C₃ alkyl chain of X is optionally substituted by 1 or 2 substituents that may be oxo or thio, and wherein the alkyl chain may be interrupted by 1 to 2 O, N, S, SO or SO₂ groups; and wherein
- the heteroaromatic or aromatic ring systems of Ar" are optionally substituted by one or two alkyl, alkylen, alkynesubstituents and/or alkoxysubstituents and wherein the mono-, bi- or tricyclic aromatic or heteroaromatic ring systems may be further substituted by electron withdrawing groups directly bound to an aromatic C-atom.

3. A compound according to claim 1 or 2, wherein
- the optional alkyl, alkylen, alkynesubstituents and/or alkoxysubstituents of Ar, Ar' and Ar" are selected from the group consisting of oxo or hydroxyl,
and wherein the alkyl, alkylen, alkynesubstituents and/or alkoxysubstituents of Ar, Ar' and Ar" may be interrupted by 1 - 5 oxygen atoms, preferably the subtituents are polyethylenglycol-moieties,
and wherein further the alkyl, alkylen, alkynesubstituents and/or alkoxysubstituents of Ar, Ar' and Ar" may comprise C₃-C₆ cycloalkyl moieties, and wherein the optional electron withdrawing groups of Ar, Ar' and Ar" are selected from the group consisting of -CN or CF₃; and wherein
- B is optionally selected from the group consisting of direct bond, CONH-CH₂CO, CO-(CH₂)₂CO, (CH₂)ₙCO, O(CH₂)ₙCO with n=1 to 10, (CH=CH)CO, 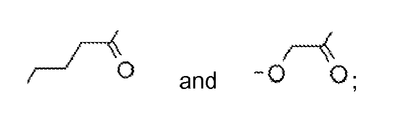 and wherein
- X is optionally selected from the group consisting of direct bond, OCH₂, NHCO, CH2O, CONH, NHCS, or CSNH.

4. A compound according to claims 1 - 3, wherein
- Ar is optionally selected from the group consisting of propylpyrimidin-2-yl, ethoxyphenyl, (CH₂CH₂O)₃phenyl, alkylphenyl, alkoxyphenyl, N-alkylindolyl, and alkylpyridyl, 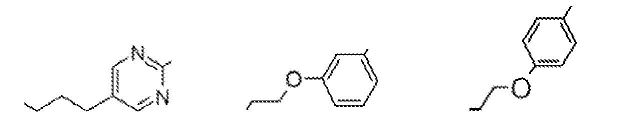 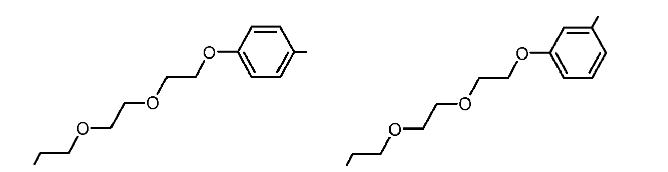 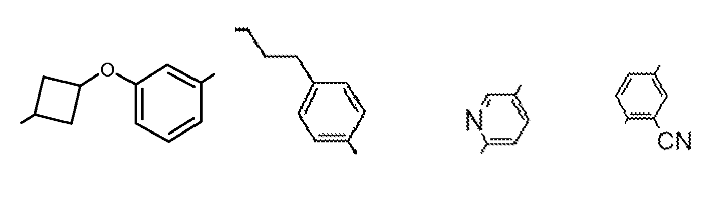 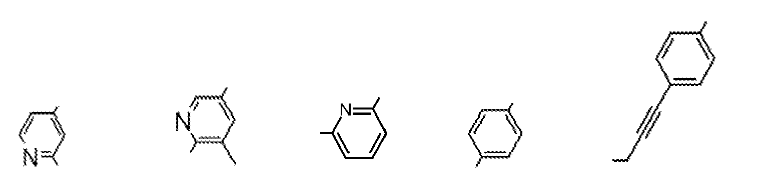 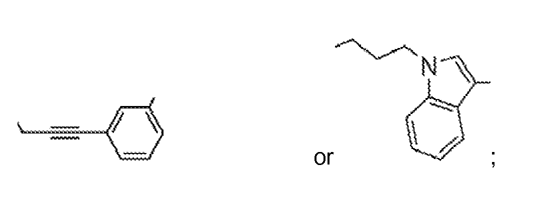 and wherein
- Ar' is optionally selected from the group consisting of propylpyrimidin-2-yl, ethoxyphenyl, (CH₂CH₂O)₃phenyl, alkylphenyl, alkoxyphenyl, N-alkylindolyl, phenyl, benzofuranyl, indolyl and alkylpyridyl; and wherein
- Ar" is optionally selected from the group consisting of phenyl, 1-phenyl, 1-naphthyl, 2-naphthyl, and all respective heterocyles thereof.

5. A compound, selected from the group consisting of

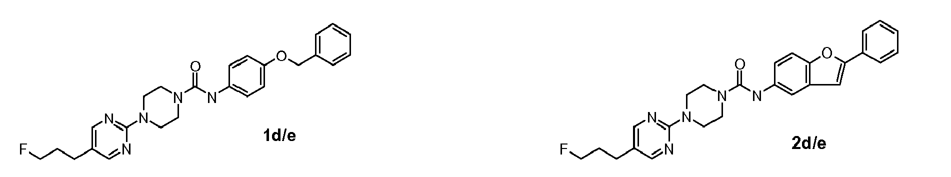

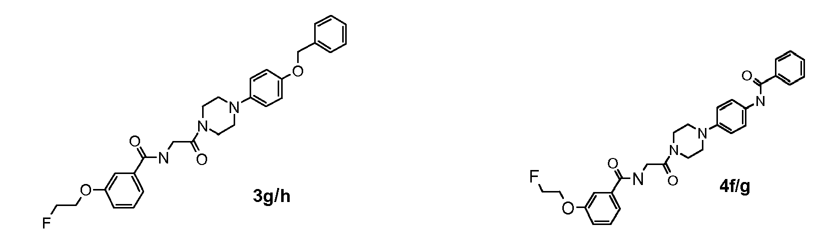

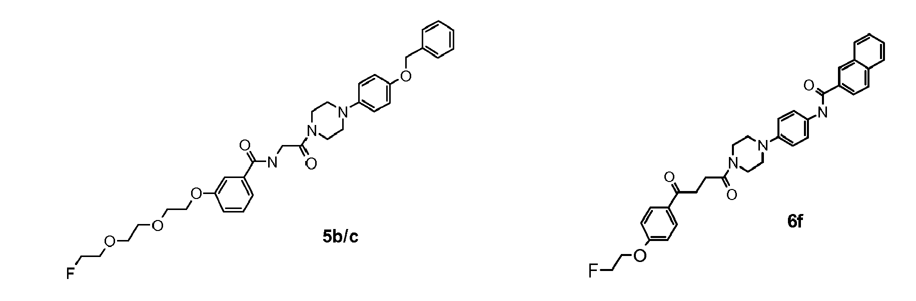

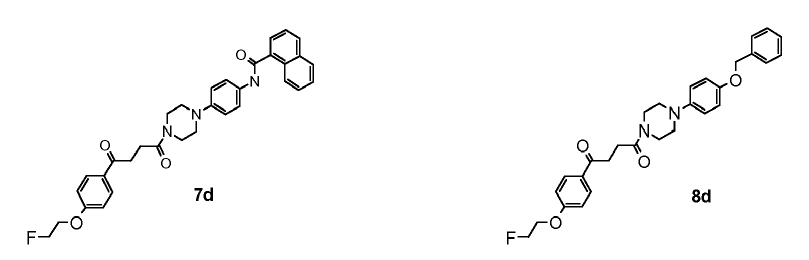

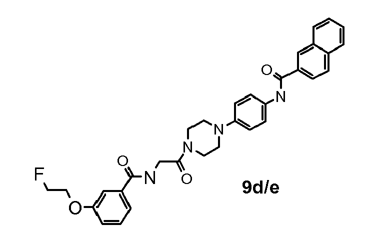

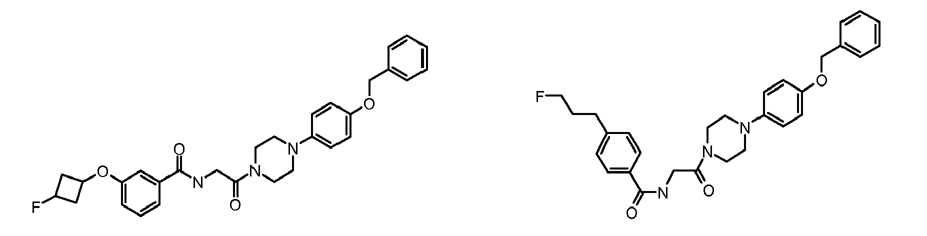

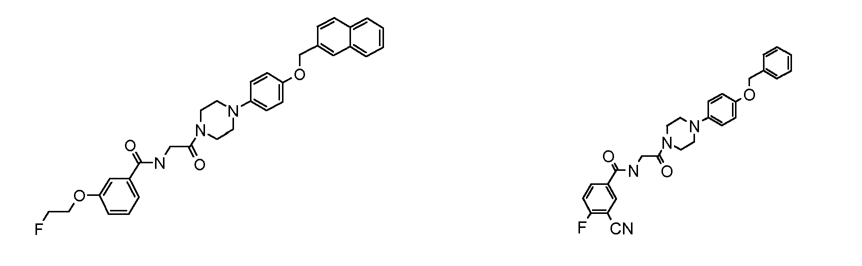

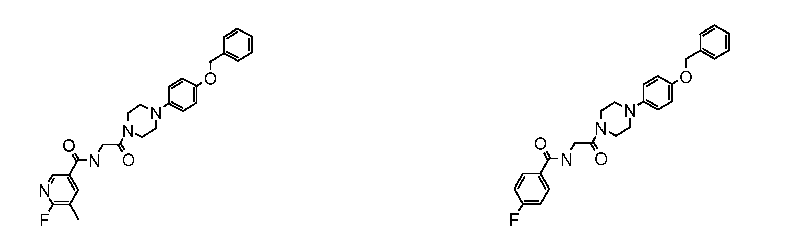

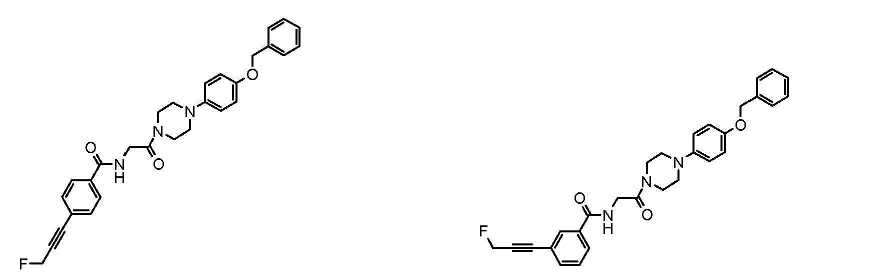

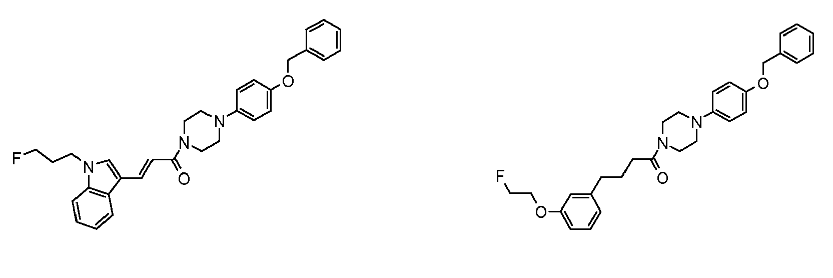

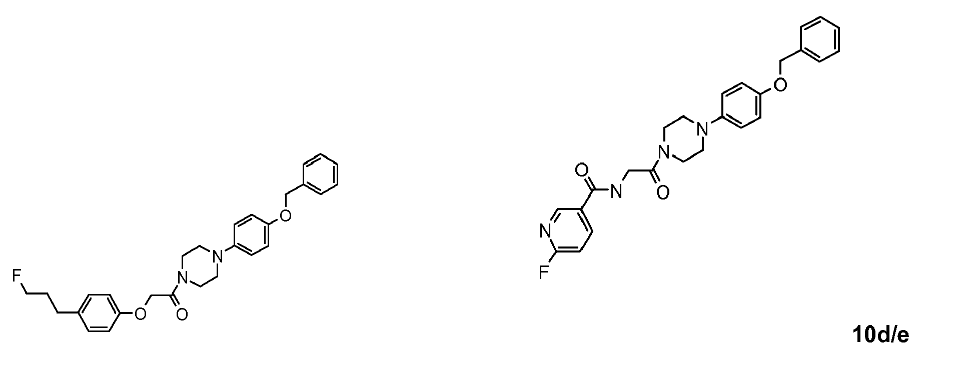

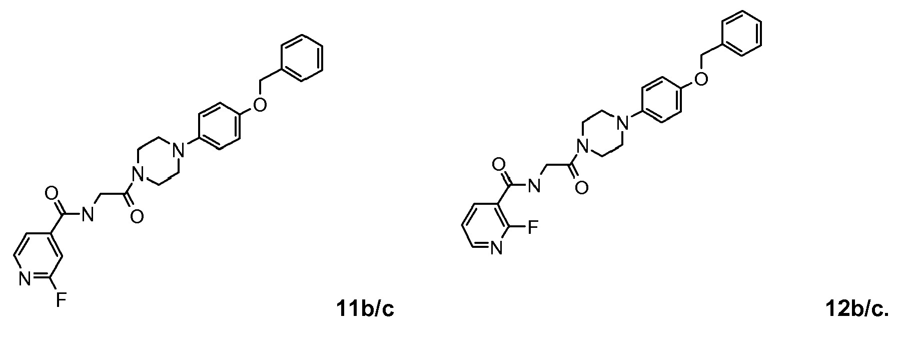

6. A compound according to claim 5, wherein F has the meaning of ¹⁸F.

7. A compound according to claim 1, 2, 3 or 4 containing a detectable label, such as a radioactive nuclide or a fluorescent label.

8. A compound according to claim 7, wherein the detectable label is ¹⁸F.

9. A compound according to claims 1 - 8 as a diagnostic compound.

10. A compound according to claims 1 - 5 or 7 as a medicament.

11. A compound according to claim 8, as a diagnostic compound for a disease selected from the group consisting of Alzheimer's disease, a neurodegenerative disorder, or an amyloidosis.

12. A compound according to claim 10 as a medicament for treating a disease selected from the group consisting of Alzheimer's disease, a neurodegenerative disorder, or an amyloidosis.

13. A method for the preparation of a fluorinated compound according to claims 1 - 8, the method comprising reacting a suitable precursor molecule with a fluorinating agent.

14. A method for treating or preventing a disorder selected from the group consisting of Alzheimer's disease, a neurodegenerative disorder, or an amyloidosis in a mammal, this method comprising administering a therapeutically effective amount of a compound according to claims 1 - 5 or 7 to said mammal.

15. Use of a compound according to claims 1 - 5 or 7 in the treatment of a disease selected from the group consisting of Alzheimer's disease, a neurodegenerative disorder, or an amyloidosis in a mammal, wherein a therapeutically effective amount of said compound is administered to said mammal.

16. A method for diagnosing a disease in a mammal selected from the group consisting of Alzheimer's disease, a neurodegenerative disorder, or an amyloidosis, this method comprising administering to said mammal a compound according to claim 6 or 8.

17. The method of claim 16, this method comprising imaging of said mammal and detecting the imaging signal.

18. The method of claim 17, where said imaging is performed using an imaging method selected from the group consisting of PET, SPECT, MR-spectroscopy, and MR-tomography.

19. A method according to claims 16 - 18, wherein the effect of a therapy is monitored.

20. A method for diagnosing or therapy monitoring of a disease selected from the group consisting of Alzheimer's disease, a neurodegenerative disorder, or an amyloidosis in a mammal, said method comprising analyzing *in vitro* a sample of said mammal,
wherein said mammal or sample has been treated with a compound according to claims 6 or 8.

21. The method of claim 20, wherein the sample is cerebrospinal fluid.

22. A kit comprising a compound according to claims 1 - 8.
